(19) Europäisches Patentamt
European Patent Office
Office européen des brevets

(11) **EP 2 458 369 B1**

(12) **EUROPÄISCHE PATENTSCHRIFT**

(45) Veröffentlichungstag und Bekanntmachung des Hinweises auf die Patenterteilung:
**23.07.2014 Patentblatt 2014/30**

(51) Int Cl.:
***G01N 21/17*** *(2006.01)* ***A61B 5/145*** *(2006.01)*

(21) Anmeldenummer: **10192470.2**

(22) Anmeldetag: **24.11.2010**

(54) **Erfassungsvorrichtung zum Erfassen eines Blutbildparameters**

Recording device for recording a blood count parameter

Dispositif de détection pour la détection d'un paramètre d'hémogramme

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**30.05.2012 Patentblatt 2012/22**

(73) Patentinhaber:
• **eesy-id GmbH**
  **82166 Gräfelfing (DE)**
• **Friedrich-Alexander-Universität Erlangen-Nürnberg**
  **91054 Erlangen (DE)**

(72) Erfinder: **Fischer, Georg**
**90425 Nürnberg (DE)**

(74) Vertreter: **Klinski, Robert et al**
**PATENTSHIP**
**Patentanwaltskanzlei**
**Elsenheimerstraße 65**
**80687 München (DE)**

(56) Entgegenhaltungen:
**WO-A1-2010/131029       GB-A- 2 428 093**
**US-A1- 2005 085 725       US-A1- 2006 025 664**
**US-A1- 2009 275 814**

**Beschreibung**

Die vorliegende Erfindung betrifft das Gebiet der Erfassung einer Konzentration eines Blutbestandteiles, beispielsweise einer Konzentration von Blutzucker.

Zur Bestimmung eines Blutbildparameters wie beispielsweise einer Konzentration eines Blutbestandteils kann invasiv Blut entnommen werden. Der Blutbildparameter kann dann unter Verwendung des entnommenen Bluts anhand standardisierter Teststreifen bestimmt werden, deren elektrische Widerstandswerte von der Konzentration des Blutbestandteiles, beispielsweise Blutzuckers, abhängen. Der jeweilige elektrische Widerstandswert kann beispielsweise unter Verwendung eines Blutzuckermessgerätes, das eine Gleichstrom-Widerstandsmessung zur Erfassung eines elektrischen Widerstandswertes eines Testreifens durchführt, erfasst werden. Der Widerstandswert kann anhand eines an sich bekannten Zusammenhanges zwischen einer Blutzuckerkonzentration und einem Widerstandswert in eine Blutzuckerkonzentration umgerechnet werden. Zur Erzielung einer hohen Erfassungsgenauigkeit ist jeder Teststreifen mit Kalibrierdaten, beispielsweise mit einem Referenzwiderstandswert oder mit einer entsprechenden Codierung, versehen, wodurch Variationen von Eigenschaften der Teststreifen ausgeglichen werden können. Nachteilig an invasiven Verfahren ist jedoch die Notwendigkeit der Blutentnahme und somit einer Verletzung eines Patienten. Außerdem ist eine kontinuierliche Erfassung einer Konzentration eines Blutbestandteils, um beispielsweise dessen Tagesgangkurve zu ermitteln, aufwändig. Darüber hinaus kann mittels des invasiven Verfahrens eine Zeitverzögerung zwischen einer Nahrungsaufnahme und beispielsweise einem Anstieg des Blutzuckers nicht genau erfasst werden. Ferner kann insbesondere bei einer geringen Konzentration des Blutzuckers in Blut ein Zeitpunkt der Insulinabgabe an den Patienten nicht genau bestimmt werden.

Zur nichtinvasiven Bestimmung eines Blutbildparameters wie beispielsweise einer Stoffkonzentration oder einer Stoffzusammensetzung in Blut können mikrowellenspektroskopische Verfahren eingesetzt werden. Die Mikrowellenspektroskopie zur Erfassung von Blutbildparametern basiert auf einer Einkopplung eines Mikrowellensignals in ein blutdurchströmtes Gewebe und einer Erfassung einer frequenzabhängigen Absorption eingekoppelter Mikrowellenenergie.

Die Druckschrift US 2006/0025664 A1 betrifft eine nicht-invasive Messung von Blutzuckerkonzentrationen. Ein Millimeterwellenerzeuger erzeugt elektromagnetische Wellen in einem bestimmten Frequenzband und führt die Wellen über einen Wellenleiter zu einem Testobjekt führt. Leistungsdetektoren erfassen die Leistung der eingestrahlten Welle und der vom Testobjekt reflektierten Welle. Die Blutzuckerkonzentration wird anhand der Verschiebung eines einzigen Absorptionsminimums bestimmt.

Die Druckschrift D2, WO 2010/131029 A1, betrifft eine nicht-invasive Überwachung von Blutbestandteilen. Das beschriebene Gerät umfasst eine Schaltung zum Bereitstellen eines Stromes mit einer Mikrowellenfrequenz, einen Sensor zum Einführen der Mikrowellenenergie in den Körper, einen Detektor zum Erfassen eines reflektierten oder transmittierten Signals, so dass ein Spektrum aufgenommen werden kann.

In der Druckschrift von Andreas Caduff et al., "Non-invasive glucose monitoring in patients with Type 1 diabetes: A multi-sensor system combining sensors for dielectric and optical characterization of skin", Biosensors and Bioelectronics 24 (2009) 2778-2784, ist eine Mehrelektrodenanordnung zur mikrowellenbasierten Bestimmung eines Blutbildparameters beschrieben. Die Mehrelektrodenanordnung umfasst mehrere Elektrodenpaare mit unterschiedlichen Elektrodenabständen, durch welche unterschiedliche Eindringtiefen von Mikrowellensignalen realisiert werden können. Die Erfassung des Blutbildparameters erfolgt mittels einer Impedanzmessung, also mittels einer Eintormessung, und ist daher fehleranfällig bei etwaigen Impedanzfehlanpassungen. Aufgrund von unterschiedlichen Eindringtiefen ist mitunter keine Unterscheidung zwischen kapillarem und venösem Blut möglich, was die Messergebnisse verfälschen kann. Eine Messung eines Blutbildparameters an venösem Blut ist im Allgemeinen genauer als eine Messung des Blutbildparameters an kapillarem Blut, weil beispielsweise Blutzuckeränderungen in kapillarem Blut verzögert gegenüber venösem Blut sind.

In den Druckschriften von Buford Randal Jean et al., "A microwave frequency sensor for non-invasive blood-glucose measurement, SAS 2008 - IEEE Sensors Applications Symposium, Atlanta, GA, February 12 - 14, 2008, and M. McClung, Calibration methodology for a microwave non-invasive glucose sensor, Master Thesis, Baylor University, Mai 2008, ist eine weitere Elektrodenanordnung zur Bestimmung einer Blutzuckerkonzentration beschrieben. Dabei wird ausgenutzt, dass die dielektrischen Eigenschaften von Blut von einem Blutzuckergehalt abhängen. Durch Anpressen eines Daumens auf den Mikrowellensensor wird eine Änderung der Dielektrizitätszahl des Daumens durch eine Verstimmung eines Resonators gemessen. Durch das Anpressen des Daumes wird jedoch Blut verdrängt, was zu einer Verfälschung der Messergebnisse führen kann. Des Weiteren können die Messungen nicht kontinuierlich durchgeführt werden. Die Auswertung der Messdaten zur Bestimmung des Blutzuckergehalts hängt zudem von dem jeweiligen Patienten ab und ist daher bei anderen Patienten nicht reproduzierbar. Darüber hinaus ist mit diesem Verfahren die Eindringtiefe der Mikrowellenleistung nicht steuerbar, so dass eine Unterscheidung zwischen kapillarem und venösem Blut nicht möglich ist. Ferner wird die Änderung der Dielektrizitätszahl auf der Basis einer Eintormessung durchgeführt, welche anfällig bezüglich Fehlanpassungen ist.

Es ist die Aufgabe der vorliegenden Erfindung, ein effizientes Konzept zur mikrowellenbasierten, nicht-invasiven Bestimmung eines Blutbildparameters, insbesondere einer Konzentration von Blutzucker, in einem durch ein Blutgefäß fließenden Blut zu schaffen.

Diese Aufgabe wird durch die Merkmale der unabhängigen Ansprüche gelöst. Vorteilhafte Weiterbildungsformen sind Gegenstand der abhängigen Ansprüche.

Die Erfindung basiert auf der Erkenntnis, dass ein Blutbildparameter durch eine Erfassung einer Frequenzverschiebung einer oder mehrerer Absorptionslinien eines Blutbestandteils bestimmt werden kann. Ist der zu bestimmende Blutbildparameter beispielsweise eine Konzentration von Blutzucker in Blut, so ist eine Frequenzverschiebung von Absorptionslinien einer Zucker enthaltenden Wasserlösung ein Maß für die Konzentration des Blutzuckers, d.h. für den Blutzuckerspiegel. Durch eine Beobachtung einer Frequenzverschiebung bei mehreren Absorptionslinien kann die Zuverlässigkeit der Bestimmung des Blutparameters zudem weiter erhöht werden. Die Erfassung des Blutbildparameters auf der Basis der Frequenzverschiebung der Absorptionslinien basiert auf der weiteren Erkenntnis, dass die Viskosität einer Wasserlösung mit zunehmender Zuckerkonzentration sich ändern kann, wodurch beispielsweise die Absorptionslinien einer Wasser-Zucker-Lösung bei geringeren Frequenzen als Absorptionslinien von reinem Wasser auftreten können. Auf diese Weise kann durch eine Erfassung einer Frequenzverschiebung von einer oder mehrerer Absorptionslinien in einem Frequenzbereich von beispielsweise 2 bis 12 GHz die Konzentration des Blutzuckers erfasst werden.

Die Erfindung basiert auf der weiteren Erkenntnis, dass ein Blutgefäß wie beispielsweise eine Vene oder eine Arterie, das dieses Blutgefäß umgebende Fettgewebe sowie die darüber liegende Hautschicht als ein dielektrisches Wellenleitersystem aufgefasst werden können. Bei einer Anregung eines derartigen dielektrischen Wellenleitersystems können daher unterschiedliche Moden bzw. Wellentypen angeregt werden, beispielsweise eine transversal-elektromagnetische (TEM) Welle oder transversal-elektrische (TE) Welle oder transversal-magnetische (TM) Welle oder eine HE-Welle. Bei einer TE-Welle existiert eine in Ausbreitungsrichtung weisende, von Null verschiedene Komponente des Magnetfeldes. Bei einer TM-Welle existiert hingegen eine in Modenausbreitungsrichtung weisende, von Null verschiedene Komponente eines elektrischen Feldes. In Abhängigkeit von einer Hochfrequenzanregung können daher in einem das Blutgefäß und die Hautschicht umfassenden dielektrischen Wellenleitersystem unterschiedliche, auch in Blutflussrichtung ausbreitungsfähige Moden angeregt werden, wodurch eine genaue Erfassung eines Blutbildparameters möglich ist.

Gemäß einem Aspekt betrifft die Erfindung eine Erfassungsvorrichtung zum Erfassen eines Blutbildparameters von Blut in einem Blutgefäß, mit einem Sender, welcher ausgebildet ist, in das Blutgefäß ein erstes Sendesignal mit einer ersten Frequenz und ein zweites Sendesignal mit einer zweiten Frequenz einzukoppeln, einem Empfänger, welcher ausgebildet ist, ein erstes Empfangssignal bei der ersten Frequenz und ein zweites Empfangssignal bei der zweiten Frequenz zu empfangen, und einem Verlustdetektor, welcher ausgebildet ist, eine erste Verlustgröße auf der Basis des ersten Sendesignals und des ersten Empfangssignals bei der ersten Frequenz zu bestimmen, und eine zweite Verlustgröße auf der Basis des zweiten Sendesignals und des zweiten Empfangssignals bei der zweiten Frequenz zu bestimmen, sowie einem Prozessor, welcher ausgebildet ist, eine erste Frequenzverschiebung der ersten Verlustgröße relativ zu einer ersten Referenzverlustgröße zu bestimmen, eine zweite Frequenzverschiebung der zweiten Verlustgröße relativ zu einer zweiten Referenzverlustgröße zu bestimmen, und den Blutbildparameter auf der Basis der ersten Frequenzverschiebung und der zweiten Frequenzverschiebung zu bestimmen. Die Verlustgrößen können beispielsweise elektromagnetische Verlustgrößen sein.

Die erste Referenzverlustgröße und die zweite Referenzverlustgröße können beispielsweise im Vorfeld anhand von Experimenten oder Messungen bestimmt werden. Handelt es sich bei der ersten Verlustgröße und der zweiten Verlustgröße beispielsweise um Absorptionen elektromagnetischer Energie, so können die erste Referenzverlustgröße und die zweite Referenzverlustgröße beispielsweise Absorptionen elektromagnetischer Energie durch eine Referenzwasserlösung mit einer Konzentration eines Blutbestandteils, beispielsweise Blutzuckers, sein.

Gemäß einer Ausführungsform betrifft die Erfindung eine Erfassungsvorrichtung, die ferner einen Speicher zum Bereitstellen der ersten Referenzverlustgröße und der zweiten Referenzverlustgröße umfasst. Die Referenzverlustgrößen können, wie vorstehend ausgeführt, im Vorfeld beispielsweise im Labor ermittelt und gespeichert werden.

Gemäß einer Ausführungsform betrifft die Erfindung eine Erfassungsvorrichtung, wobei die erste Verlustgröße eine Absorptionslinie einer Wasserlösung mit einem Blutbestandteil bei der ersten Frequenz und wobei die zweite Verlustgröße eine Absorptionslinie der Wasserlösung bei der zweiten Frequenz sein kann. Handelt es sich bei dem Blutbestandteil um Blutzucker, so entstehen bei unterschiedlichen Frequenzen Absorptionslinien, mit beispielsweise Absorptionsmaxima oder Absorptionsminima, deren Frequenzlagen von der Konzentration des Blutzuckers abhängen. Aus den Frequenzverschiebungen der Absorptionslinien gegenüber Referenzabsorptionslinien, welche durch die vorstehend genannten Referenzverlustgrößen gebildet sein können, kann der Blutbildparameter bestimmt werden.

Gemäß einer Ausführungsform betrifft die Erfindung eine Erfassungsvorrichtung, wobei die erste Verlustgröße und die zweite Verlustgröße einen frequenzabhängigen Verlauf von Absorptionen einer Wasserlösung mit einem Blutbestandteil definieren, und wobei die erste Referenzgröße und die zweite Referenzgröße einen frequenzabhängigen Verlauf von Absorptionslinien der Wasserlösung mit einer Referenzkonzentration des Blutbestandteils definieren.

Gemäß einer Ausführungsform betrifft die Erfindung eine Erfassungsvorrichtung, wobei die erste Verlustgröße ein Absorptionsminimum oder ein Absorptionsmaximum in einem die erste Frequenz umfassenden ersten Frequenzbereich ist, und wobei die zweite Verlustgröße ein Absorptionsminimum oder ein Absorptionsmaximum in einem die zweite Frequenz umfassenden zweiten Frequenzbereich ist.

Gemäß einer Ausführungsform betrifft die Erfindung eine Erfassungsvorrichtung, wobei der Blutbildparameter eine Konzentration eines Blutbestandteils, insbesondere Zuckers wie Glucose, oder Laktats oder Milchsäure oder Sauerstoffs, in Blut ist.

Gemäß einer Ausführungsform betrifft die Erfindung eine Erfassungsvorrichtung, wobei die erste Frequenz und die zweite Frequenz jeweils in einem Frequenzbereich zwischen 1 GHz und 15 GHz liegen.

Gemäß einer Ausführungsform betrifft die Erfindung eine Erfassungsvorrichtung, wobei der Verlustdetektor ausgebildet ist, die erste Verlustgröße und die zweite Verlustgröße durch eine Zweitormessung zu bestimmen.

Gemäß einer Ausführungsform betrifft die Erfindung eine Erfassungsvorrichtung, wobei der Verlustdetektor einen Netzwerkanalysator, insbesondere einen vektoriellen oder einen skalaren Netzwerkanalysator, oder einen Leistungsdetektor umfasst.

Gemäß einer Ausführungsform betrifft die Erfindung eine Erfassungsvorrichtung, wobei der Verlustdetektor ausgebildet ist, zur Bestimmung der ersten Verlustgröße und der zweiten Verlustgröße jeweils einen Vorwärtstransmissionsfaktor $S_{21}$ und/oder einen Eingangsreflexionsfaktor $S_{11}$ zu bestimmen.

Gemäß einer Ausführungsform betrifft die Erfindung eine Erfassungsvorrichtung, wobei der Verlustdetektor ausgebildet ist, die erste Verlustgröße und die zweiten Verlustgröße jeweils auf der Basis der folgenden Formel zu bestimmen:

$$P_{Verlust} = 1 - \left| S_{11} \right|^2 - \left| S_{21} \right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

Gemäß einer Ausführungsform betrifft die Erfindung eine Erfassungsvorrichtung, wobei der Sender zum Einkoppeln des ersten Sendesignals oder des zweiten Sendesignals zumindest eine Sendeantenne, insbesondere eine Dipolantenne, eine Rahmenantenne oder eine Patch-Antenne, umfasst, und wobei der Empfänger zum Empfangen des ersten Empfangssignals und des zweiten Empfangssignals zumindest eine Empfangsantenne, insbesondere eine Dipolantenne, eine Rahmenantenne oder eine Patch-Antenne, welche von der Sendeantenne beabstandet sein kann, umfasst.

Gemäß einer Ausführungsform betrifft die Erfindung eine Erfassungsvorrichtung, wobei der Sender ausgebildet ist, das erste Sendesignal oder das zweite Sendesignal als eine Mode oder einen Wellentyp, insbesondere als eine transversal-elektrische (TE) Welle oder als eine transversal-magnetische (TM) Welle oder als eine transversal-elektromagnetische (TEM) Welle oder als eine HE-Welle, in das Blutgefäß einzukoppeln, insbesondere longitudinal oder transversal bezüglich eines Verlaufes des Blutgefäßes einzukoppeln.

Gemäß einer Ausführungsform betrifft die Erfindung eine Erfassungsvorrichtung, wobei der Sender ausgebildet ist, das erste Sendesignal und das zweite Sendesignal sukzessive, insbesondere mittels eines Sendesignalgenerators oder eines durchstimmbaren Oszillators, oder gleichzeitig, insbesondere mittels des das erste Sendesignal und das zweite Sendesignal umfassenden Breitbandsignals, in das Blutgefäß einzukoppeln.

Gemäß einem weiteren Aspekt betrifft die Erfindung ein Verfahren zum Erfassen eines Blutbildparameters von Blut in einem Blutgefäß, mit den Schritten des Einkoppelns eines ersten Sendesignals mit einer ersten Frequenz in das Blutgefäß, des Einkoppelns eines zweiten Sendesignals mit einer zweiten Frequenz in das Blutgefäß, des Empfangens eines ersten Empfangssignals bei der ersten Frequenz, des Empfangens eines zweiten Empfangssignals bei der zweiten Frequenz, des Ermittelns einer ersten Verlustgröße auf der Basis des ersten Sendesignals und des ersten Empfangssignals bei der ersten Frequenz, des Ermittelns einer zweiten Verlustgröße auf der Basis des zweiten Sendesignals und des zweiten Empfangssignals bei der zweiten Frequenz, des Bestimmens einer ersten Frequenzverschiebung der ersten Verlustgröße relativ zu einer ersten Referenzverlustgröße, des Bestimmens einer zweiten Frequenzverschiebung der zweiten Verlustgröße relativ zu einer zweiten Referenzverlustgröße, und des Bestimmens des Blutbildparameters auf der Basis der ersten Frequenzverschiebung und der zweiten Frequenzverschiebung.

Weitere Verfahrensschritte ergeben sich unmittelbar aus der Funktionalität der Erfassungsvorrichtung zum Erfassen eines Blutbildparameters.

Weitere Ausführungsbeispiele werden Bezug nehmend auf die beiliegenden Zeichnungen näher erläutert. Es zeigen:

Fig. 1          ein Blockdiagramm einer Erfassungsvorrichtung;

Fig. 2          ein Modell eines Querschnitts eines menschlichen Unterarms;

Fig. 3A, 3D          Antennen;

| Fig. 4A | eine elektrische Dipolantenne; |
| Fig. 4B | eine Erregeranordnung; |
| Fig. 5A bis 5B | Erregeranordnungen; |
| Fig. 6A | eine Schleifenantenne; |
| Fig. 6B | eine Erregeranordnung; |
| Fig. 7 | eine Erregeranordnung; |
| Fig. 8 | eine Erregeranordnung; |
| Fig. 9 | eine Erregeranordnung; |
| Fig. 10 | eine Erregeranordnung; |
| Fig. 11 | ein prinzipielles Schaltbild einer Erfassungsvorrichtung; |
| Fig. 12 | eine Frequenzverschiebung eines Absorptionsmaximums; |
| Fig. 13 | Transmissionsverhalten; |
| Fig. 14 | Frequenzverschiebungen; |
| Fig. 15 | ein Diagramm eines Verfahrens zum Erfassen eines Blutbildparameters; |
| Fig. 16 | ein prinzipielles Schaltbild einer Erfassungsvorrichtung; |
| Fig. 17 | ein Blockdiagramm eines Armbandes; |
| Fig. 18 | ein Blockdiagramm eines Ausschnittes eines Armbandes; |
| Fig. 19 | ein Blockdiagramm eines Armbandes; und |
| Fig. 20 | ein Blockdiagramm einer Anordnung der Elektroden der Erfassungsvorrichtung. |

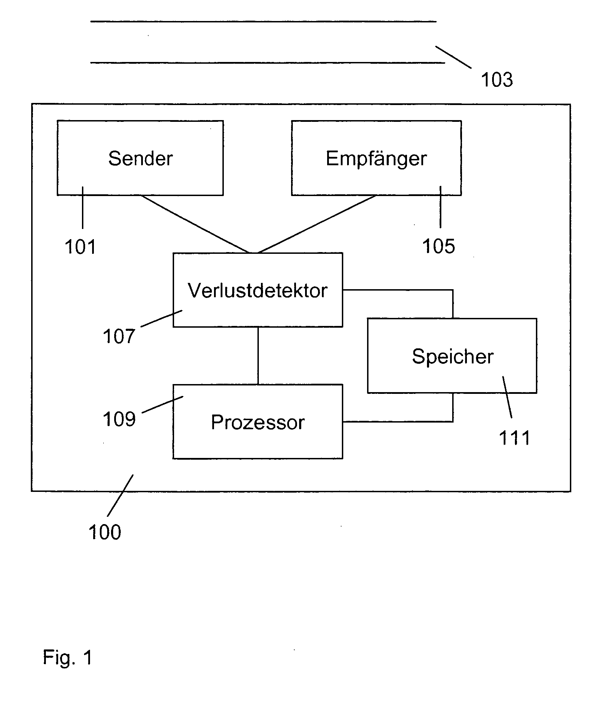

Fig. 1 zeigt ein Blockdiagramm einer Erfassungsvorrichtung 100 zum Erfassen eines Blutbildparameters wie beispielsweise einer Konzentration von Blutzucker. Die Erfassungsvorrichtung 100 umfasst einen Sender 101, welcher ausgebildet ist, in das in Fig. 1 schematisch dargestellte Blutgefäß 103 ein erstes Sendesignal mit einer ersten Frequenz und ein zweites Sendesignal mit einer zweiten Frequenz einzukoppeln. Das erste Sendesignal und das zweite Sendesignal können beispielsweise gemeinsam ein Breitbandsignal ergeben. Der Sender 101 kann ausgebildet sein, das erste Sendesignal und das zweite Sendesignal hintereinander, beispielsweise durch einen Frequenz-Sweep, auszusenden. Hierzu kann der Sender 101 eine oder mehrere Sendeantennen, welche beispielsweise als Dipolantennen oder Rahmenantennen oder Patch-Antennen ausgebildet sein können, aufweisen.

Die Erfassungsvorrichtung 100 umfasst ferner einen Empfänger 105, welcher ausgebildet ist, ein erstes Empfangssignal bei der ersten Frequenz und ein zweites Empfangssignal bei der zweiten Frequenz zu empfangen. Hierzu kann der Empfänger 105 eine oder mehrere Empfangsantennen aufweisen.

Die Erfassungsvorrichtung 100 umfasst ferner einen Verlustdetektor 107, welcher beispielsweise mit dem Sender 101 und dem Empfänger 105 gekoppelt und vorgesehen ist, eine erste Verlustgröße auf der Basis des ersten Sendesignals und des ersten Empfangssignals sowie eine zweite Verlustgröße auf der Basis des zweiten Sendesignals und des zweiten Empfangssignals zu bestimmen.

Die Erfassungsvorrichtung umfasst ferner einen Prozessor 109, welcher mit dem Verlustdetektor 107 gekoppelt und vorgesehen ist, eine erste Frequenzverschiebung der ersten Verlustgröße relativ zu einer ersten Referenzverlustgröße und eine zweite Frequenzverschiebung der zweiten Verlustgröße relativ zu einer zweiten Referenzverlustgröße zu bestimmen. Der Prozessor 109 kann ferner ausgebildet sein, den Blutbildparameter auf der Basis der beiden Frequenzverschiebungen zu bestimmen.

Die Erfassungsvorrichtung 100 kann ferner einen Speicher 111 aufweisen, auf welchen beispielsweise der Prozessor 109 und optional der Verlustdetektor 107 zugreifen können. In dem Speicher 111 sind beispielsweise die erste und die zweite Referenzverlustgröße oder eine Mehrzahl von Referenzverlustgrößen abgelegt. Bei den Referenzverlustgrößen kann es sich beispielsweise um Absorptionen oder Absorptionslinien einer Wasserlösung mit einem Blutbestandteil, beispielsweise Blutzucker, handeln. Die auf der Basis der Frequenzverschiebungen erfassten Verlustgrößen können frequenzverschobene Absorptionen oder Absorptionslinien sein, so dass auf der Basis der Frequenzverschiebungen der Blutbildparameter, wie beispielsweise eine Konzentration von Blutzucker, ermittelt werden kann.

Die in Fig. 1 dargestellte Erfassungsvorrichtung 100 nutzt die Erkenntnis aus, dass ein Blutgefäß, eine Hautschicht sowie ein das Blutgefäß umgebendes Fettgewebe beispielsweise eines menschlichen Unterarms als ein dielektrisches Wellenleitersystem aufgefasst werden können. Der Aufbau eines menschlichen Unterarms ist beispielsweise in Netter, F. N., Atlas der Anatomie, Thieme Verlag, 2006, beschrieben. Ein menschlicher Unterarm besteht aus zwei Knochen, welche von einem Muskelgewebe umgeben sind. Um das Muskelgewebe herum sind oberflächliche Venen, d.h. Blutgefäße, verteilt. Die Knochen, das Muskelgewebe sowie die Venen sind durch ein Fettgewebe ummantelt, welches durch obere Hautschichten bedeckt ist. Die oberflächlichen Venen sind relativ nahe an den oberen Hautschichten angeordnet und von diesen durch das Fettgewebe separiert. Werden beispielsweise der in Fig. 1 dargestellte Sender 101 und der Empfänger 105 auf die obere Hautschicht aufgelegt, so kann der Sender 101 dazu eingesetzt werden, in das durch ein Blutgefäß, ein Fettgewebe sowie eine Hautschicht gebildete dielektrische Wellenleitersystem beispielsweise eine transversal-elektrische (TE) Welle oder eine transversal-magnetische (TM) Welle einzukoppeln. Dabei können die Hautschicht sowie das Fettgewebe als ein Filmwellenleiter verstanden werden.

Wird beispielsweise ein Mikrowellenmesskopf, wie er für die Bestimmung einer komplexen Dielektrizitätszahl von Materialien benutzt werden kann, verwendet, so kann dadurch das Stoffgemisch bestehend aus Haut, Fettgewebe und Venen charakterisiert werden.

Um einen Blutbildparameter zu erfassen, ist es vorteilhaft, im Wesentlichen nur das venöse Blut zu erfassen. Hierzu kann der Sender 101 ausgebildet sein, das Sendesignal in der Gestalt einer elektromagnetischen Welle direkt in das Blutgefäß 103 einzukoppeln. Der Sender 101 sowie der Empfänger 105 können jeweils eine Mehrzahl von Antennen aufweisen, so dass zur Einkopplung der elektromagnetischen Welle in das Blutgefäß 103 und zur Auskopplung einer elektromagnetischen Welle aus dem Blutgefäß jeweils diejenige Sendeantenne und diejenige Empfangsantenne ausgewählt werden können, welche mit den geringsten Koppelverlusten verbunden sind.

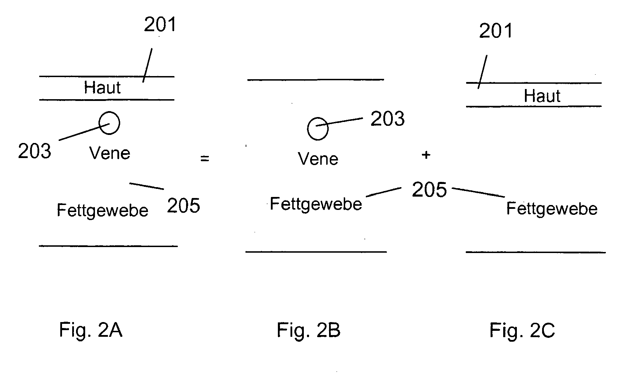

In den Figuren 2A bis 2C ist ein vereinfachtes Modell eines Querschnitts eines menschlichen Unterarms, z.B. eines Handgelenks, wie es beispielsweise für Feldsimulationen bzw. zur Modellierung eines dielektrischen Wellenleitersystems benutzt werden kann. Wie in Fig. 2A dargestellt, umfasst das Modell eine Hautschicht 201, ein Blutgefäß 203 sowie ein das Blutgefäß 203 umgebendes Fettgewebe 205. Das in Fig. 2A dargestellte Modell bildet ein dielektrisches Wellenleitersystem umfassend den in Fig. 2B dargestellten dielektrischen Wellenleiter sowie den in Fig. 2C dargestellten elektrischen Filmwellenleiter.

Der in Fig. 2B dargestellte dielektrische Wellenleiter umfasst das Blutgefäß 203 sowie das dieses umgebende Fettgewebe 205. Der dielektrische Filmwellenleiter aus Fig. 2C umfasst die Hautschicht 201 sowie das Fettgewebe 205. Für die Hautschicht 201, das Fettgewebe 205 sowie für das Blutgefäß 203 kann jeweils ein unterschiedliches dispersives, d.h. frequenzabhängiges, Verhalten der jeweiligen komplexen Dielektrizitätszahl angesetzt werden. Das oben liegende Blutgefäß 203 wird dabei als ein dielektrischer Wellenleiter interpretiert, in dem je nach Frequenz verschiedene Moden bzw. Wellentypen, beispielsweise eine TE-Welle, eine TM-Welle, eine TEM-Welle oder eine HE-Welle, ausbreitungsfähig sein können. Zu dem Wellenleitermechanismus in dem dielektrischen Wellenleiter kommt ein weiterer Wellenleitermechanismus in der Gestalt des in Fig. 2C dargestellten Filmwellenleiters hinzu, der durch die obere Hautschicht 201 gebildet ist.

Eine Sendeantenne des Senders 101 sowie eine Empfangsantenne des Empfängers 105 können bevorzugt derart ausgestaltet sein, dass sie dezidiert Mikrowellenleistung in das Blutgefäß 203 einkoppeln und diese beispielsweise nach einigen Zentimetern wieder auskoppeln. Das Blutgefäß 203 dient dabei als eine Messstrecke und ist somit als ein verteiltes Element und nicht mehr als ein konzentriertes Element zu sehen. Bevorzugt wird die Messung der Verlustgrößen auf der Basis einer Zweitormessung durchgeführt. Dabei können insbesondere bei einer Ankopplung der Erfassungsvorrichtung an ein Handgelenk Primärmoden in dem dielektrischen Wellenleiter 2B angeregt werden, so dass eine Anregung von Filmwellenleitermoden in dem Filmwellenleiter 2C vermieden wird, so dass die Erfassung des Blutbildparameters genauer durchgeführt werden kann.

Um Primärmoden im dielektrischen Wellenleitersystem anzuregen, kann berücksichtigt werden, dass je nach gewählter Frequenz eines Sendesignals unterschiedliche Moden dominant sein können. Bevorzugt sind Modentypen, die eine Konzentration der Felder in dem Blutgefäß 203 aufweisen, solchen Moden vorzuziehen, bei denen die Felder in der Hautschicht 201 konzentriert sind. Aufgrund der dielektrischen Eigenschaften des in Fig. 2B dargestellten dielektrischen Wellenleiters zeigt sich, dass für bestimmte Modentypen longitudinale Komponenten $E_{longitudinal}$, $H_{longitudinal}$ in Ausbreitungsrichtung, d.h. in Richtung eines Blutgefäßverlaufs, stärker als die transversalen Komponenten $E_{transversal}$, $H_{transversal}$, d.h. quer zum Blutgefäßverlauf, sind. Bevorzugt werden daher in dem zu erfassenden Frequenzbereich diejenigen Moden angeregt, welche eine maximale Einkopplung der Mikrowellenleistung in das Blutgefäß 203 ermöglichen.

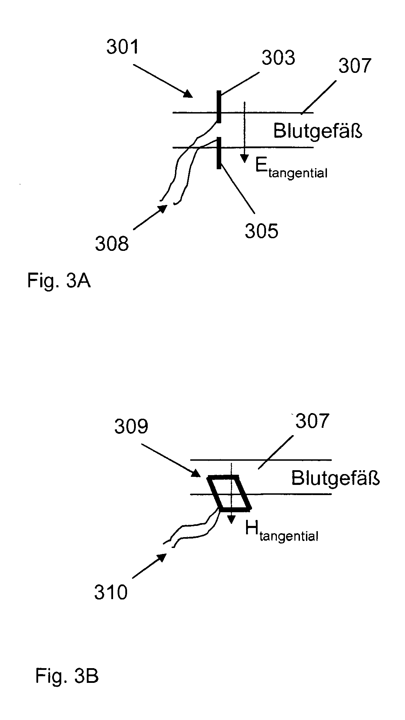

In den Figuren 3A bis 3D sind beispielhaft einige Antennen dargestellt, welche als Sendeantennen, d.h. Erreger, oder auch als Empfangsantennen eingesetzt werden können.

Die in Fig. 3A dargestellte Antenne 301 ist als ein elektrischer Dipol mit einem ersten Antennenabschnitt 303 und einem zweiten Antennenabschnitt 305 ausgeführt Die Antennenabschnitte 303 und 305 sind voneinander beabstandet und beispielsweise quer zu einem Verlauf eines Blutgefäßes 307 angeordnet. Eine Anregung der Antenne 301 kann durch Zuleitungen 308 erfolgen. Ein derart angeordneter elektrischer Dipol kann beispielsweise ein elektrisches Feld $E_{tangential}$ erzeugen, das quer zum Verlauf des Blutgefäßes bzw. zur Blutflussrichtung zeigt.

In Fig. 3B ist eine Antenne 309 dargestellt, welche eine Rahmenantenne sein kann. Die Rahmenantenne kann beispielsweise eine Viereckform oder eine runde Form aufweisen. Bei der in Fig. 3B dargestellten Anordnung der Rahmenantenne 309 bezüglich des Blutgefäßes 307 wird beispielsweise ein magnetisches Feld $H_{tangential}$ angeregt, das quer zum Verlauf des Blutgefäßes 307 bzw. quer zur Blutflussrichtung zeigt. Eine Anregung der Antenne 309 kann durch Zuleitungen 310 erfolgen.

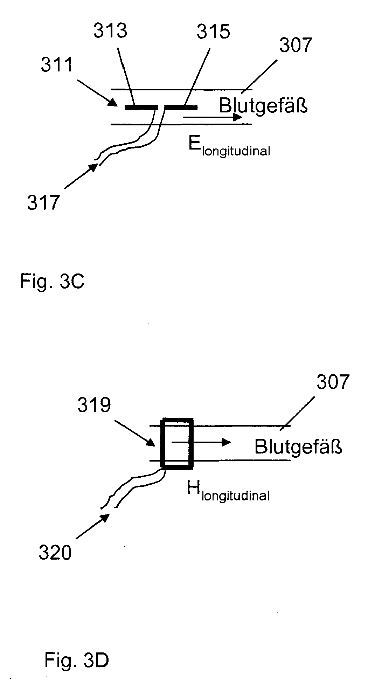

In Fig. 3C ist eine Antenne 311 dargestellt, welche einen elektrischen Dipol mit einem ersten Antennenabschnitt 313 und einem zweiten Antennenabschnitt 315 bildet. Die Antennenabschnitte 313 und 315 sind voneinander beabstandet und werden mittels der in Fig. 3C dargestellten Zuleitungen 317 angeregt. Der durch die Antenne 311 gebildete elektrische Dipol ist derart bezüglich des Verlaufs des Blutgefäßes 307 angeordnet, dass die Abschnitte 313 und 315 parallel zum Verlauf des Blutgefäßes 307 angeordnet sind. Dadurch wird ein in Richtung des Verlaufs des Blutgefäßes weisendes elektrisches Feld mit der Feldkomponente $E_{longitudinal}$ angeregt.

Fig. 3D zeigt eine Rahmenantenne 319, welche beispielsweise in der Gestalt eines viereckigen oder runden Rahmens, welcher eine Schleifenantenne bildet, beispielsweise als eine Patch-Antenne, gebildet sein kann. Die Rahmenantenne 319 wird mittels Zuleitungen 320 angeregt und ist wie es in Fig. 3D dargestellt ist, bezüglich des Verlaufs des Blutgefäßes 307 bzw. bezüglich der Blutflussrichtung derart angeordnet, dass das magnetische Feld eine in Richtung des Verlaufs des Blutgefäßes 307 weisende Komponente $H_{longitudinal}$ aufweist.

Der jeweils zu messende Frequenzbereich richtet sich beispielsweise danach, welche Spektrallinien, d.h. welche Absorptionslinien, zu erfassen sind. Es können beispielsweise die charakteristischen Absorptionslinien eines Stoffes oder auch ein Effekt beobachtet werden, den ein bestimmter Blutbestandteil auf die Absorptionslinien von Wasser bzw. von einer Wasserlösung mit einer Konzentration des Blutbestandteils aufweist.

Bei den in den Figuren 3A bis 3D dargestellten Antennen handelt es sich entweder um elektrische Dipole oder um magnetische Rahmenantennen. Darüber hinaus können auch Patch-Antennen eingesetzt werden. Elektrische Dipole erzeugen dominant ein elektrisches Feld in der Achse des elektrischen Dipols. Diese Achse kann entweder, wie es in Fig. 3A dargestellt ist, tangential zum Blutgefäß 307 bzw. zur Blutflussrichtung, oder wie es in Fig. 3C dargestellt ist, in Richtung des Blutgefäßes 307 bzw. in Blutflussrichtung ausgerichtet sein. Falls primär ein magnetisches Feld erzeugt werden soll, so kann eine Rahmenantenne als Erreger eingesetzt werden. Ist ein Flächenvektor auf der von dem die Rahmenantenne bildenden Rahmen aufgespannten Fläche quer zu dem Blutgefäß 307 bzw. quer zur Blutflussrichtung ausgerichtet, so ist auch das magnetische Feld quer zu dem Blutgefäß 307 ausgerichtet, wie es in Fig. 3B dargestellt ist. Zeigt der Flächenvektor hingegen in Richtung des Blutgefäßes 307, so ist auch das Magnetfeld in Richtung des Blutgefäßes 307 ausgerichtet, wie es beispielsweise in Fig. 3B dargestellt ist. Aus der Wahl eines der in den Figuren 3A bis 3D dargestellten Erregers ergibt sich dann beispielsweise die dominant angeregte Mode bzw. Wellentyp.

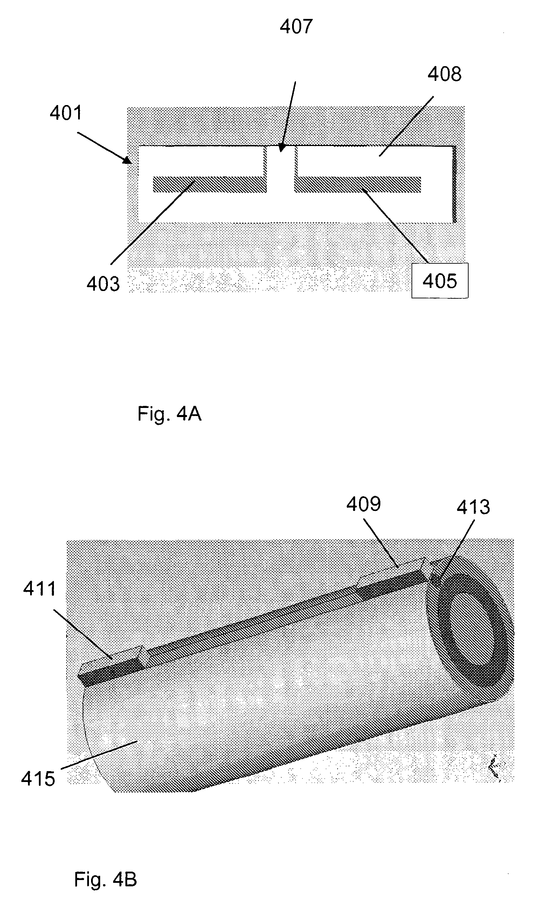

Fig. 4A zeigt eine elektrische Dipolantenne 401, welche als eine Sendeantenne oder als eine Empfangsantenne eingesetzt werden kann. Die elektrische Dipolantenne 401 umfasst Dipolantennenabschnitte 403 und 405, welche in oder auf einem Substrat 408 angeordnet sind und mittels Zuleitungen 407 angeregt werden können. Die Dipolantenne 401 kann als Sendeantenne oder als Empfangsantenne eingesetzt werden.

Fig. 4B zeigt eine Erregeranordnung einer Sendeantenne 409 eines Senders und einer Empfangsantenne 411 eines Empfängers in Richtung eines Verlaufs eines Blutgefässes 413 unterhalb einer Hautschicht 415. Die Sendeantenne 409 und die Empfangsantenne 411 sind beispielsweise elektrische Dipolantennen gemäß Fig. 4A. Bei der in Fig. 4B dargestellten Anordnung wird ein elektrisches Feld mit einer Feldkomponente in Richtung des Verlaufs des Blutgefässes 413, bzw. in Blutflussrichtung erzeugt.

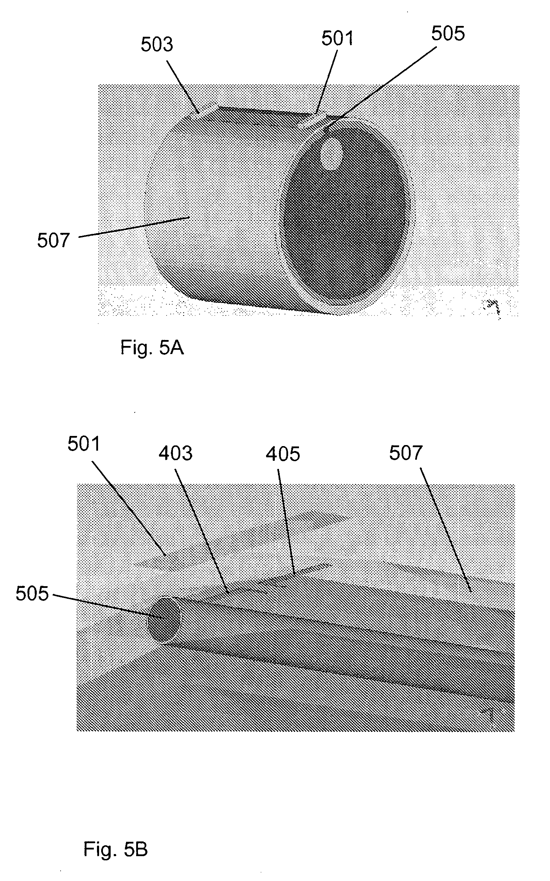

Fig. 5A zeigt eine Erregeranordnung umfassend eine Sendeantenne 501 eines Senders und eine Empfangsantenne 503 eines Empfängers quer zur Ausbreitungsrichtung eines Blutgefäßes 505, d.h. quer zur Blutflussrichtung, das unter einer Hautschicht 507 liegt. Die Sendeantenne 501 und die Empfangsantenne 503 können jeweils beispielsweise durch die in Fig. 4A dargestellte elektrische Dipolantenne gebildet sein. In Fig. 5B ist die Anordnung der Dipolantennenabschnitte 403 und 405 bezüglich der Blutflussrichtung genauer dargestellt.

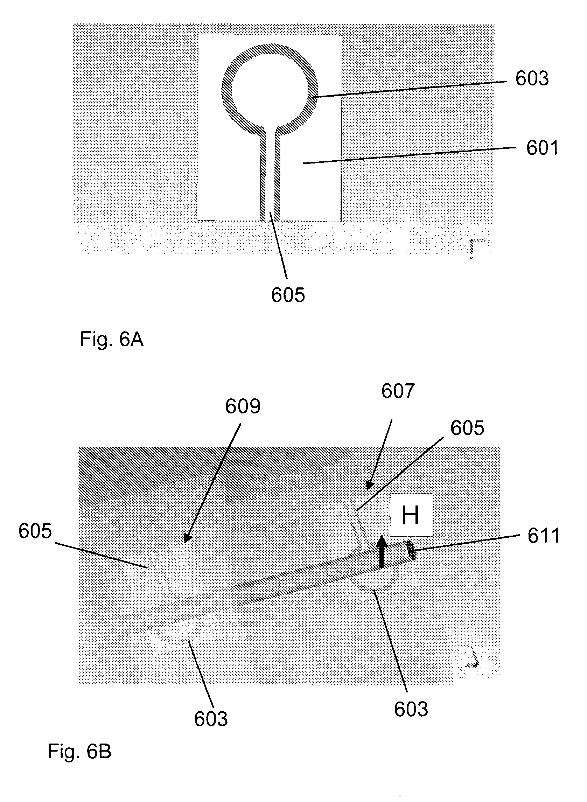

Fig. 6A zeigt eine Schleifenantenne 601 mit einem kreisförmigen Rahmen 603 und Zuleitungen 605 zum Anregen des kreisförmigen Rahmens 603. Die Schleifenantenne 601 kann beispielsweise als eine Sendeantenne oder als eine Empfangsantenne eingesetzt werden. Der kreisförmigen Rahmen 603 sowie die Zuleitungen 605 können in einem Substrat angeordnet werden.

Fig. 6B zeigt eine Erregeranordnung mit einer Sendeantenne 607 eines Senders und einer Empfangsantenne 609 eines Empfängers, welche als Schleifenantennen gemäß Fig. 6A gebildet sein können. Die Schleifenantennen 607, 609 sind beispielsweise derart angeordnet, dass die kreisförmigen Rahmen 603 oberhalb eines Blutgefäßes 611 angeordnet sind, wobei die Zuleitungen 605 quer zum Verlauf des Blutgefäßes 611, d.h. quer zur Blutflussrichtung, zeigen. Dadurch wird senderseitig ein Magnetfeld mit einer quer zum Verlauf des Blutgefäßes 611 weisenden Komponente H des Magnetfeldes erzeugt.

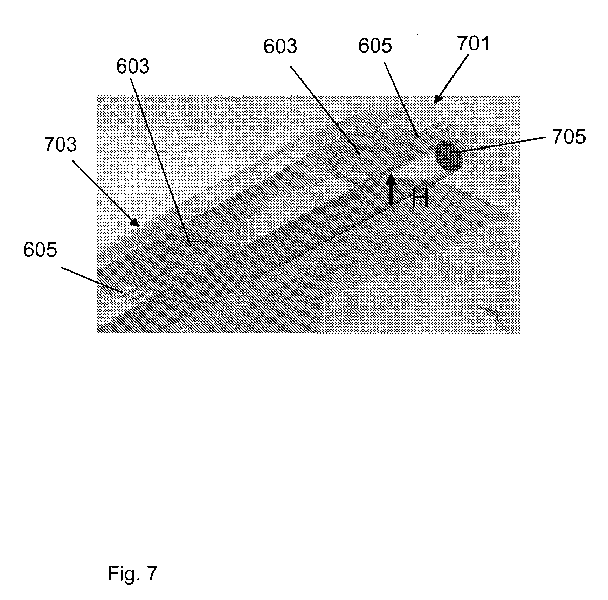

Fig. 7 zeigt eine Erregeranordnung einer Sendeantenne 701 eines Senders und einer Empfangantenne 703 eines Empfängers bezüglich eines Blutgefässes 705. Die Sendeantenne 701 und die Empfangsantenne 703 können beispielsweise Schleifenantennen mit der in Fig. 6A dargestellten Form sein. Sie sind beispielsweise derart angeordnet, dass die kreisrunden Rahmen 603 jeweils oberhalb des Blutgefässes 705 angeordnet sind und dass die Zuleitungen 605 voneinander weisend parallel zum Verlauf des Blutgefäßes 705 verlaufen. Dadurch wird eine senkrecht zum Verlauf des Blutgefäßes 705 weisende magnetische Feldkomponente H erzeugt, welche in Richtung einer Normalen der durch den kreisrunden Rahmen 603 aufgespannten Fläche zeigt.

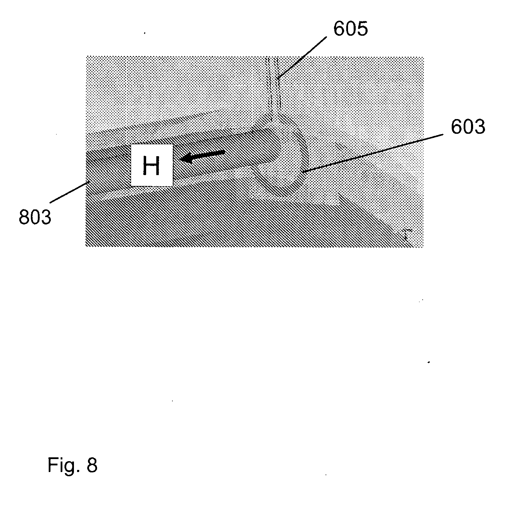

Fig. 8 zeigt eine Erregeranordnung mit einer Sendeantenne 801 eines Senders, welche beispielsweise die in Fig. 6A dargestellte Form einer Schleifenantenne aufweist. Die Sendeantenne 801 ist bezüglich eines Blutgefässes 803 beispielsweise derart angeordnet, dass eine Normale der durch den Rahmen 603 aufgespannten Fläche in Richtung des Verlaufes des Blutgefäßes 803 zeigt. Eine derartige Anordnung kann beispielsweise bei einem Knick des Blutgefäßes 803 realisiert werden. Dadurch wird eine in Richtung des Verlaufes des Blutgefäßes 803 weisende magnetische Feldkomponente H erzeugt.

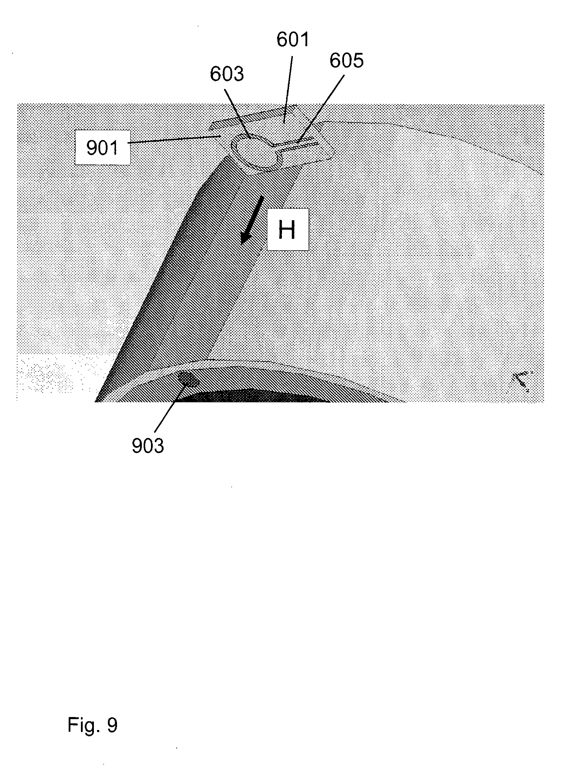

Fig. 9 zeigt eine Erregeranordnung mit einer Sendeantenne 601, welche beispielsweise eine Schleifenantenne mit der in Fig. 6A dargestellten Form ist und in oder auf einem Substrat 901, beispielsweise Kunststoffsubstrat, angeordnet sein kann. Die Sendeantenne 601 ist oberhalb eines Blutgefässes 903 derart angeordnet, dass eine Normale der durch den kreisförmigen Rahmen 603 aufgespannten Fläche in Richtung des Verlaufes des Blutgefäßes 903 zeigt. Dadurch wird ein magnetisches Feld mit einer in Richtung des Verlaufes des Blutgefäßes 903, d.h. in Blutflussrichtung, weisenden magnetischen Feldkomponente H erzeugt.

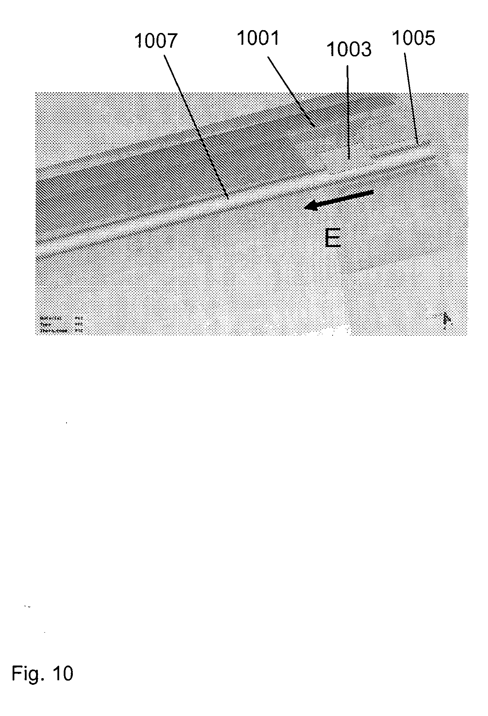

Fig. 10 zeigt eine Erregeranordnung mit einer Sendeantenne 1001, welche eine Patch-Antenne mit einer Patch-Antennenfläche 1003 und Zuleitungen 1005 sein kann. Die Patch-Antennenfläche 1003 ist beispielsweise oberhalb eines Blutgefäßes 1007 angeordnet, wodurch ein elektrisches Feld mit einer in Richtung eines Verlaufs des Blutgefäßes 1007, d.h. in Blutflussrichtung, weisenden elektrischen Feldkomponente E erzeugt wird.

Gemäß einer weiteren Ausführungsform ist der Verlustdetektor 107 ausgebildet, beispielsweise eine skalare oder eine vektorielle Messung oder eine Leistungsmessung durchzuführen. Zur Bestimmung der Verlustgrößen kann eine einfache spektroskopische Messung durchgeführt werden, bei der der Betrag des Messparameters S21 erfasst wird.

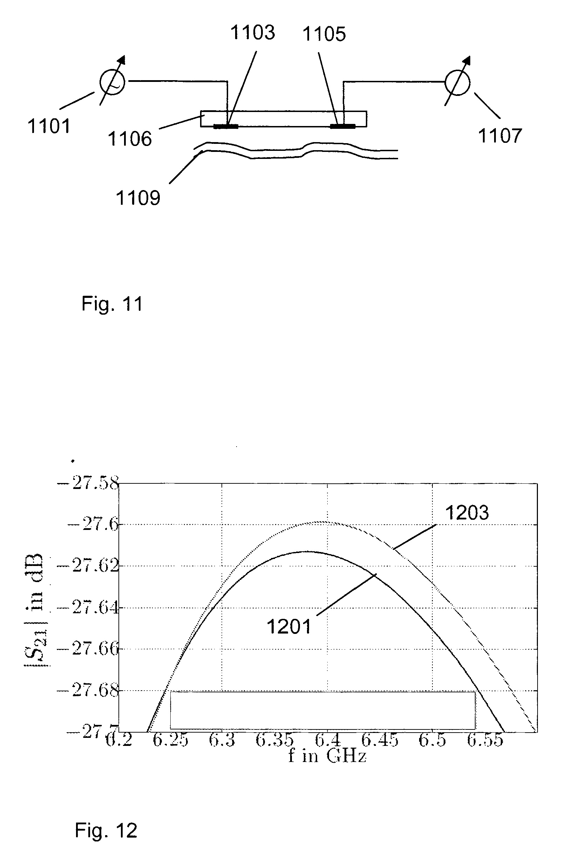

Die Messung von |S21| kann beispielsweise mittels der in Fig. 11 dargestellten Erfassungsvorrichtung durchgeführt werden. Die Erfassungsvorrichtung umfasst einen Sender mit einem Sendesignalgenerator 1101, welcher ein durchstimmbarer Oszillator sein kann. Ein Ausgang des Sendesignalgenerators 1101 ist mit einer Sendeantenne 1103 verbunden. Die Erfassungsvorrichtung umfasst ferner einen Empfänger mit einer Empfangsantenne 1105, deren Ausgang mit einem Verlustdetektor 1107 verbunden ist. Der Verlustdetektor kann beispielsweise einen Leistungsdetektor umfassen. Wie in Fig. 11 dargestellt sind die Sendeantenne 1103 und die Empfangsantenne 1105 oberhalb eines Blutgefäßes 1109 angeordnet. Der Sender kann Merkmale des Senders 101, der Empfänger kann Merkmale des Empfängers 105 und der Verlustdetektor 1107 kann Merkmale des Verlustdetektors 107 aufweisen.

Durch eine weitere Messung eines Betrags des Messparameters von S11 kann die Genauigkeit bei der Bestimmung der Verlustgrößen, d.h. der Verluste in dem Wellenleiter, noch weiter gesteigert werden. Die Verlustgrößen können beispielsweise auf der Basis der folgenden Formel bestimmt werden:

$$P_{Verlust} = 1 - \left| S_{11} \right|^2 - \left| S_{21} \right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

Zum Erfassen des Blutbildparameters, beispielsweise einer Konzentration von Blutzucker, können beispielsweise Frequenzverschiebungen von Absorptionslinien einer Wasserlösung mit Zucker untersucht werden. Fig. 12 zeigt beispielsweise eine Frequenzverschiebung eines Absorptionsmaximums 1201 bei einer ersten Blutzuckerkonzentration im Vergleich zu einer Frequenzverschiebung eines Absorptionsmaximums 1203 bei einer zweiten Blutzuckerkonzentration, welche höher als die erste Blutzuckerkonzentration. Dabei wurde als Verlustgröße beispielhaft eine Transmission um 6 GHz erfasst.

Die Frequenzverschiebung des Absorptionsmaximums kann als ein Maß für einen Blutbildparameter, beispielsweise für einen Blutzuckerspiegel, aufgefasst werden. Durch eine Beobachtung von Frequenzverschiebungen bei mehreren Absorptionen einer Wasserlösung mit Zucker kann die Messzuverlässigkeit noch weiter erhöht werden.

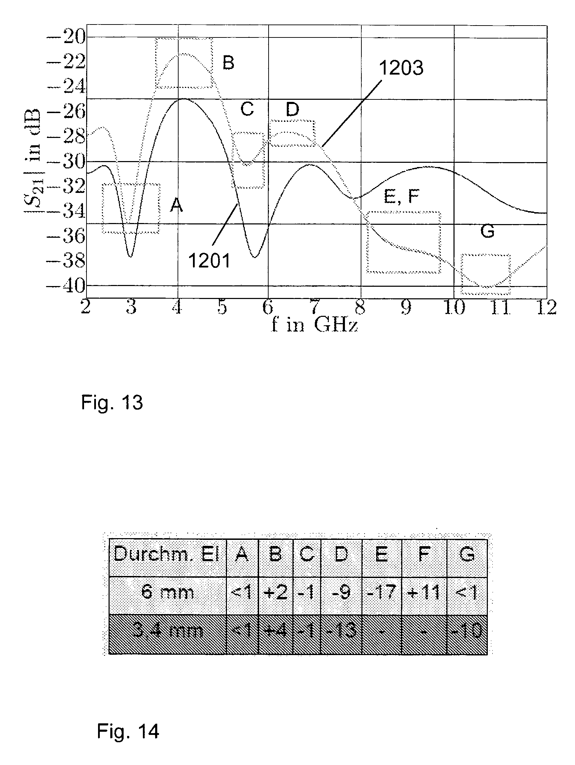

Fig. 13 zeigt Transmissionsverhalten von venösem Blut in einem Handgelenk. Dabei verdeutlichen die Verläufe 1301 und 1303 unterschiedliche Frequenzlagen von Absorptionslinien bei unterschiedlichen Blutzuckerkonzentrationen. Zur Erfassung des Blutbildparameters, wie beispielsweise der Konzentration des Blutzuckers, können beispielsweise gezielt Frequenzverschiebungen der Absorptionen A, B, C, D, E, F und G erfasst werden. So kann beispielsweise für jede Frequenz eines Absorptionsmaximums und/oder eines Absorptionsminimums eine Verschiebung in Richtung höherer oder niedriger Frequenzen je nach Blutzuckerspiegel beispielsweise in einem Frequenzbereich zwischen 2 GHz und 12 GHz beobachtet werden.

Fig. 14 zeigt beispielhaft Frequenzverschiebungen der in Fig. 13 dargestellten Absorptionen A, B, C, D, E, F und G für ein Blutgefäß mit 6 mm Durchmesser und für ein Blutgefäß mit 3,4 mm Durchmesser. Zu erkennen ist, dass die Absorptionen für eine Zuckerspiegelvariation Frequenzverschiebungen sowohl in positiver als auch in negativer Richtung aufweisen können. Die Erfassung von mehreren Absorptionen bzw. Absorptionslinien ermöglicht somit eine genauere Erfassung eines Blutbildparameters, beispielsweise des Blutzuckerspiegels.

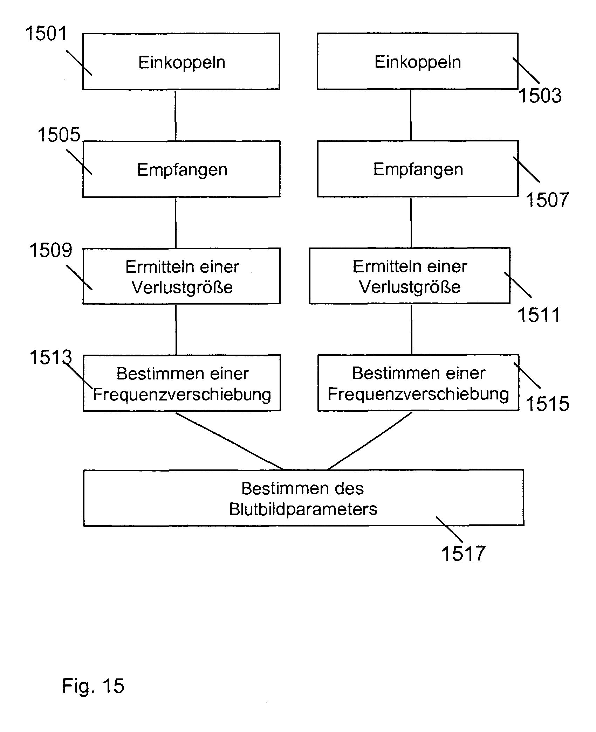

Fig. 15 zeigt ein Diagramm eines Verfahrens zum Erfassen eines Blutbildparameters von Blut in einem Blutgefäß. Das Verfahren umfasst das Einkoppeln 1501 eines ersten Sendesignals mit einer ersten Frequenz in das Blutgefäß, das Einkoppeln 1503 eines zweiten Sendesignals mit einer zweiten Frequenz in das Blutgefäß, das Empfangen 1505 eines ersten Empfangssignals bei der ersten Frequenz, das Empfangen 1507 eines zweiten Empfangssignals bei der zweiten Frequenz, das Ermitteln 1509 einer ersten Verlustgröße auf der Basis des ersten Sendesignals und des ersten Empfangssignals bei der ersten Frequenz, das Ermitteln 1511 einer zweiten Verlustgröße auf der Basis des zweiten Sendesignals und des zweiten Empfangssignals bei der zweiten Frequenz, das Bestimmen 1513 einer ersten Frequenzverschiebung der ersten Verlustgröße relativ zu einer ersten Referenzverlustgröße, das Bestimmen 1515 einer zweiten Frequenzverschiebung der zweiten Verlustgröße relativ zu einer zweiten Referenzverlustgröße, und das Bestimmen 1517 des Blutbildparameters auf der Basis der ersten Frequenzverschiebung und der zweiten Frequenzverschiebung.

Das in Fig. 15 dargestellte Verfahren kann beispielsweise durch die in Fig. 1 oder in Fig. 11 dargestellte Erfassungsvorrichtung ausgeführt werden.

Das vorstehend genannte Erfassungsprinzip kann beispielsweise in einem Armband integriert werden, das um ein Handgelenk gelegt und beispielsweise aufgepumpt werden kann. Auf diese Weise wird die Ankopplung der elektromagnetischen Wellen an den Körper reproduzierbar, da stets der gleiche Druck herrscht, welcher einen Luftspalt zwischen einer Hautschicht und den Antennen minimiert. Das vorstehend genannte Prinzip der Mikrowellenmessung ist darüber hinaus sehr robust, da nicht die Höhe der Absorptionslinien, sondern deren Frequenzverschiebung erfasst und ausgewertet wird. Die Überwachung eines Blutbildparameters wie beispielsweise eines Blutzuckergehaltes kann kontinuierlich durchgeführt werden. Damit kann eine Bestimmung einer Verzögerungszeit zwischen einer Nahrungsaufnahme und einem Blutzuckeranstieg ermöglicht werden. Darüber hinaus kann auf Variationen in einem Tagesablauf eines Patienten schneller reagiert werden. So kann beispielsweise bei einer Über- bzw. Unterzuckerung Alarm ausgelöst werden, wobei eine telemedizinische Anbindung der beispielsweise in Fig. 1 dargestellten Erfassungsvorrichtung denkbar ist.

Gemäß einer Ausführungsform können zur gezielten Mikrowelleneinkopplung in ein Blutgefäß sendeseitig mehrere Sendeantennen und empfangsseitig mehrere Empfangsantennen vorgesehen sein. Durch eine Permutation aller Antennenkombinationen kann beispielsweise dasjenige Antennenpärchen umfassend eine Sendeantenne um eine Empfangsantenne ausgewählt werden, das mit den geringsten Einkoppelverlusten verbunden ist. Das ausgewählte Antennenpärchen kann dann zur mikrowellenbasierten Erfassung des Blutbildparameters herangezogen werden.

So kann beispielsweise der in Fig. 1 dargestellte Sender 101 eine oder mehrere Sendeantennen zum Aussenden von einem oder mehreren Sendesignalen aufweisen, welche beispielsweise als Dipolantennen oder Rahmenantennen oder Patch-Antennen ausgebildet sein können. In Analogie hierzu kann der Empfänger 105 eine oder mehrere Sendeantennen zum Empfangen von einem oder mehreren Empfangssignalen aufweisen. Der Prozessor 109 ist bevorzugt ausgebildet, eine Mehrzahl von Erfassungskonfigurationen nacheinander auszuwählen. Dabei umfasst jede Erfassungskonfiguration eine einzige Sendeantenne und eine einzige Empfangsantenne, wobei die Sendeantennen voneinander beabstandet und wobei die Empfangsantennen voneinander beabstandet sein können. Bei Auswahl einer Erfassungskonfiguration erregt der Sender 101 die dazu gehörige Sendeantenne zum Aussenden eines Sendesignals, wobei der Empfänger 105 die jeweilige Empfangsantenne verwendet, um ein Empfangssignal zu empfangen.

Auf der Basis des Sendesignals und des Empfangssignals kann der Verlustdetektor 107 beispielsweise eine elektromagnetische Verlustgröße wie Energieabsorption bestimmen. Im nächsten Schritt wird eine weitere Erfassungskonfiguration zum Aussenden eines Sendesignals verwendet und es wird eine weitere Verlustgröße erfasst. Auf diese Weise werden mehrere Erfassungskonfigurationen der Reihe nach oder in beliebiger Reihenfolge zum Einkoppeln eines Sendesignals in das Blutgefäß 103 verwendet, wobei bei jeder Erfassungskonfiguration eine Verlustgröße mittels des Verlustdetektors 107 bestimmt wird. Der Prozessor 109 kann die Verlustgrößen beispielsweise vergleichen und diejenige Erfassungskonfiguration auswählen, welche mit der geringsten Verlustgröße verbunden ist. Die ausgewählte Erfassungskonfiguration wird zum Erfassen des Blutbildparameters verwendet.

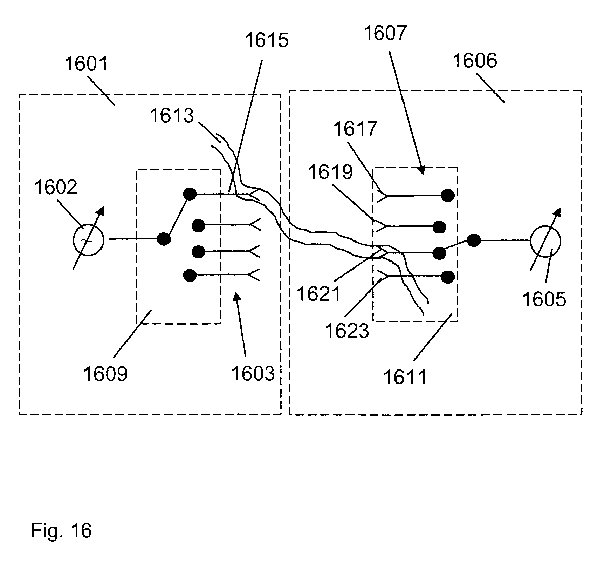

Fig. 16 zeigt eine Erfassungsvorrichtung, welche eine Ausführungsform der in Fig. 1 dargestellten Erfassungsvorrichtung 100 sein kann. Die Erfassungsvorrichtung umfasst einen Sender 1601, der beispielsweise einen durchstimmbaren Oszillator 1602 aufweisen kann, sowie eine Mehrzahl von Sendeantennen 1603. Die Erfassungsvorrichtung umfasst ferner einen Verlustdetektor 1605, welcher beispielsweise einen Leistungsdetektor aufweisen kann. Ferner ist ein Empfänger 1606 mit einer Mehrzahl von Empfangsantennen 1607 vorgesehen.

Ein Ausgang des durchstimmbaren Oszillators 1602 ist beispielsweise mittels einer Schaltmatrix 1609 mit jedem Antenneneingang beispielsweise der Reihe nach oder in beliebiger Reihenfolge schaltbar verbindbar. In Analogie hierzu ist jeder Ausgang einer Empfangsantenne der Mehrzahl der Empfangsantennen 1607 mittels einer Schaltmatrix 1611 mit dem Verlustdetektor 1605 verbindbar.

Mittels der Schaltmatrix 1611 sowie der Schaltmatrix 1609 kann beispielsweise dasjenige Paar umfassend eine Sendeantenne und eine Empfangsantenne ausgewählt werden, das eine optimale Einkopplung eines Mikrowellensignals in ein in Fig. 16 schematisch dargestelltes Blutgefäß 1613 ermöglicht. Mittels der Schaltmatrizen 1609 und 1611 werden die Antennenpaare der Reihe nach ausgewählt beginnend beispielsweise mit einer ersten Sendeantenne 1615, mittels welcher ein Sendesignal ausgesendet wird.

Die Schaltmatrizen 1609, 1611 können Schalter, beispielsweise Transistorschalter, aufweisen.

Empfangsseitig werden mittels der Schaltmatrix 1611 der Reihe nach die Empfangsantennen beginnend beispielsweise mit der Empfangsantenne 1617 zum Empfang eines entsprechenden Empfangssignals ausgewählt, wobei auf der Basis des Sendesignals und des Empfangssignals eine Verlustgröße erfasst wird. Im nächsten Schritt wird beispielsweise die Empfangsantenne 1619 ausgewählt, wobei wiederum auf der Basis des Sendesignals und eines durch die Empfangsantenne 1619 empfangenen Empfangssignals eine Verlustgröße mittels des Verlustdetektors erfasst wird. Danach wird beispielsweise die Empfangsantenne 1621 ausgewählt, wobei auf der Basis des Sendesignals und eines Empfangssignals eine weitere Verlustgröße erfasst wird. Im nächsten Schritt wird die Empfangsantenne 1623 ausgewählt und es wird auf der Basis des Sendesignals sowie eines durch die Empfangsantenne 1623 empfangenen Empfangssignals eine weitere Verlustgröße bestimmt. Im nächsten Schritt kann die Schaltmatrix 1609 beispielsweise eine weitere Sendeantenne auswählen, wobei die vorgenannten Schritte wiederholt werden können. Durch einen Vergleich der ermittelten Verlustgrößen wird beispielsweise die kleinste Verlustgröße ausgewählt. In dem in Fig. 16 dargestellten Beispiel ist beispielsweise zu erwarten, dass die Erfassungskonfiguration mit der Sendeantenne 1615 und der Empfangsantenne 1621 mit den geringsten Einkoppelverlusten behaftet ist, weil die Antennen 1615, 1621 unmittelbar oberhalb des Blutgefäßes liegen und somit eine Einkopplung eines Signals in das Blutgefäß 1613 ermöglichen. Die ausgewählte Erfassungskonfiguration kann beispielsweise zur Erfassung eines Blutbildparameters herangezogen werden. Die vorstehend beschriebenen Auswahlschritte können in beliebiger Reihenfolge durchgeführt werden. So können beispielsweise für die Sendeantenne 1615 alle oder einige der Empfangsantennen 1607 getestet werden.

Die Sendeantennen 1603 bzw. die Empfangsantennen 1607 können sich hinsichtlich ihres Ortes und/oder hinsichtlich ihrer Feldkomponente, welche dominant angeregt werden soll, unterscheiden. Durch die Schaltmatrizen 1609 und 1611 ist dabei gewährleistet, dass für die jeweils gewählte Frequenz der optimale Erregertyp, beispielsweise Schleifenanenne, elektrische Dipolantenne, Patch-Antenne oder Erregerort, ausgewählt werden kann.

Gemäß einer Ausführungsform wird die Messung breitbandig statt schmalbandig durchgeführt. Die Sendesignale können beispielsweise mittels eines Frequenzsweeps oder als Teilsignal eines breitbandigen Sendesignals in das Blutgefäß eingekoppelt werden. Durch die bevorzugt vektorielle Erfassung der S-Parameter kann nun die komplexe Dielektrizitätszahl und nicht nur deren Realteil ausgewertet werden. Durch Beobachtung von Frequenzverschiebungen von mehreren Absorptionslinien kann die Bestimmung des Blutbildparameters genauer durchgeführt werden. Bevorzugt wird dieser mittels einer Zweitormessung und nicht mittels einer Eintormessung durchgeführt.

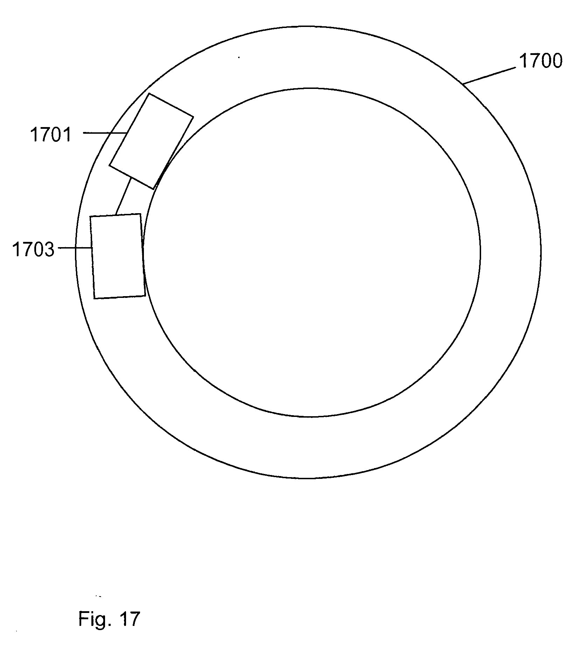

Die Fig. 17 zeigt ein Blockdiagramm eines Ausführungsbeispiels eines Armbandes 1900 mit einer Erfassungsvorrichtung 1701 und einer Einstellvorrichtung 1703. Die Erfassungsvorrichtung 1701 ist zum Erfassen eines Blutbildparameters von Blut in einem Blutgefäß des Armes eingerichtet. Ein Beispiel für den zu erfassenden Blutbildparameter ist die Glukosekonzentration in dem Blut.

Die Einstellvorrichtung 1703 ist zum Einstellen eines vorgebbaren Anpressdruckes des Armbandes 1700 auf den Arm eingerichtet. Durch Einstellen des vorgegebenen Anpressdruckes des Armbandes 1700 kann die Einstellvorrichtung 1703 reproduzierbare Erfassungen des Blutbildparameters durch die Erfassungsvorrichtung 1701 sicherstellen. Dazu ist die Einstellvorrichtung 1703 insbesondere dazu eingerichtet, den Anpressdruck des Armbandes 1700 während des Erfassens des Blutbildparameters durch die Erfassungsvorrichtung 1701 auf den vorgebbaren Anpressdruck einzustellen.

Das Armband 1700 ist insbesondere als ein aufblasbares Armband 1700 ausgebildet. Dabei hat die Einstellvorrichtung 1703 insbesondere eine Luftpumpe, welche dazu ausgebildet ist, das Armband 1700 zum Einstellen des vorgegebenen Anpressdruckes aufzublasen.

Im Detail umfasst die Erfassungsvorrichtung 1701 insbesondere Elektroden, welche zur Einkopplung zumindest eines Hochfrequenzsignals in das Blutgefäß eingerichtet sind. Das Hochfrequenzsignal ist dazu ausgebildet, einen Parameter zur Erfassung des Blutbildparameters zu liefern. Ein Beispiel für einen solchen Parameter bildet die Relaxationszeitkonstante $\tau$ des Blutbildparameters. Dabei ist die Einstellvorrichtung 1703 insbesondere dazu ausgebildet, den Anpressdruck der Elektroden auf den Arm auf den vorgegebenen Anpressdruck einzustellen.

Des Weiteren kann die Einstellvorrichtung 1703 derart ausgebildet werden, dass sie die Anpresskräfte des Armbandes 1700 während des Erfassens des Blutbildparameters durch die Erfassungsvorrichtung 1701 gleichmäßig auf den Arm verteilt. Ferner ist die Einstellvorrichtung 1703 vorzugsweise derart ausgebildet, dass sie ein gleichmäßiges Anliegen des Armbandes 1700 während des Erfassens des Blutbildparameters durch die Erfassungsvorrichtung 1701 sicherstellt.

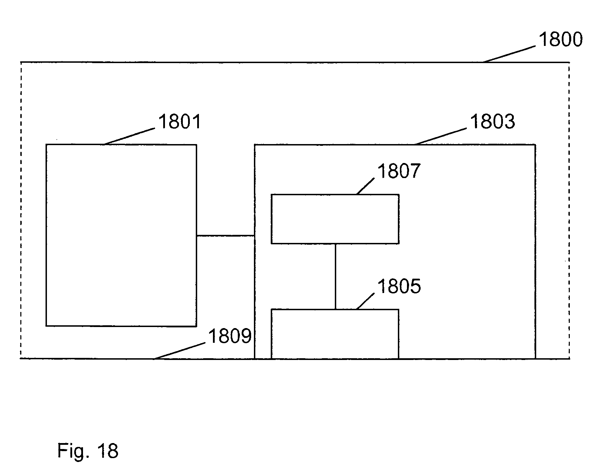

Die Fig. 18 zeigt ein Blockschaltbild eines Ausschnittes eines Ausführungsbeispiels eines Armbandes 1800. Das Armband 1800 hat eine Erfassungsvorrichtung 1801 und eine Einstellvorrichtung 1803. Die Erfassungsvorrichtung 1801 und die Einstellvorrichtung 1803 sind zumindest wie die Erfassungsvorrichtung 1701 und die Einstellvorrichtung 1703 der Fig. 17 ausgebildet. Des Weiteren hat die Einstellvorrichtung 1803 der Fig. 18 eine Sensoreinrichtung 1805 und eine Steuereinrichtung 1807. Die Sensoreinrichtung 1805 ist dazu eingerichtet, einen aktuellen Anpressdruck des Armbandes 1800 auf den Arm zu messen. In Abhängigkeit des gemessenen aktuellen Anpressdruckes stellt die Steuereinrichtung 2007 den vorgegebenen Anpressdruck auf den Arm ein.

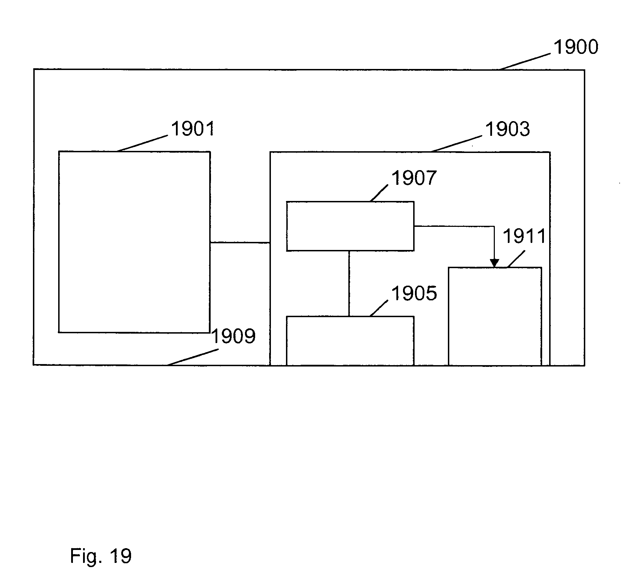

Die Fig. 19 zeigt ein Blockschaltbild eines Ausschnittes eines weiteren Ausführungsbeispiels eines Armbandes 1900. Das Armband 1900 hat eine Erfassungsvorrichtung 1901 und eine Einstellvorrichtung 1903. Die Einstellvorrichtung 1903 hat eine Sensoreinrichtung 1905, eine Steuereinrichtung 1907 und eine Luftpumpe 1911. Die Sensoreinrichtung 1905 erfasst einen aktuellen Anpressdruck des Armbandes 1900 auf den Arm. Die Steuereinrichtung 1907 stellt ein Steuersignal in Abhängigkeit des gemessenen aktuellen Anpressdruckes bereit. Mittels des bereitgestellten Steuersignals wird die Luftpumpe 1911 zum Aufblasen des Armbandes 1900 gesteuert.

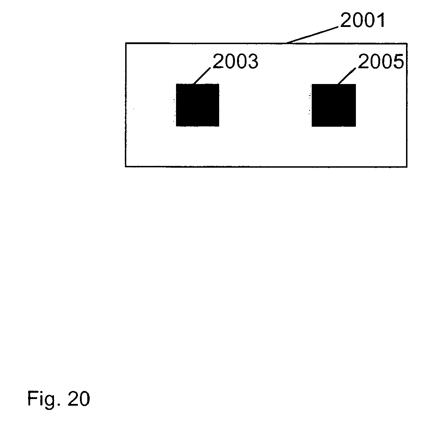

In Fig. 20 ist ein schematisches Blockdiagramm einer Anordnung 2000 der Elektroden, d.h. Antennen 2003, 2005 der Erfassungsvorrichtung zum Erfassen eines Blutbildparameters von Blut in einem Blutgefäß des Armes dargestellt.

Ohne Einschränkung der Allgemeinheit zeigt die Anordnung 2000 nur zwei Elektroden 2003 und 2005. Die Anordnung 2000 ist insbesondere Teil der Erfassungsvorrichtung und beispielsweise als eine Platte mit beispielhaften Abmessungen von 5cm auf 2cm ausgestaltet. Die Elektroden 2003, 2005 haben beispielsweise eine Grundfläche von 5mm auf 5mm. Der Abstand der Elektroden 2003, 2005 beträgt beispielsweise 1 bis 2cm. Damit wird zum einen eine genügend starke Transmission erzielt und zum anderen wird eine genügend große Eindringtiefe in den Körper sichergestellt.

**Patentansprüche**

1.  Erfassungsvorrichtung zum Erfassen eines Blutbildparameters von Blut in einem Blutgefäß, mit:

> einem Sender (101), welcher ausgebildet ist, in das Blutgefäß ein erstes Sendesignal mit einer ersten Frequenz und ein zweites Sendesignal mit einer zweiten Frequenz einzukoppeln;
> einem Empfänger (105), welcher ausgebildet ist, ein erstes Empfangssignal bei der ersten Frequenz und ein zweites Empfangssignal bei der zweiten Frequenz zu empfangen; und

einem Verlustdetektor (107), welcher ausgebildet ist:

> eine erste Verlustgröße auf der Basis des ersten Sendesignals und des ersten Empfangssignals bei der ersten Frequenz zu bestimmen; und
> eine zweite Verlustgröße auf der Basis des zweiten Sendesignals und des zweiten Empfangssignals bei der zweiten Frequenz zu bestimmen; sowie

einem Prozessor (109), welcher **dadurch gekennzeichnet ist, dass** er ausgebildet ist:

> eine erste Frequenzverschiebung der ersten Verlustgröße relativ zu einer ersten Referenzverlustgröße zu bestimmen;
> eine zweite Frequenzverschiebung der zweiten Verlustgröße relativ zu einer zweiten Referenzverlustgröße zu bestimmen; und
> den Blutbildparameter auf der Basis der ersten Frequenzverschiebung und der zweiten Frequenzverschiebung zu bestimmen.

2.  Erfassungsvorrichtung nach Anspruch 1, die ferner einen Speicher (111) zum Bereitstellen der ersten Referenzverlustgröße und der zweiten Referenzverlustgröße umfasst.

3.  Erfassungsvorrichtung nach Anspruch 1, wobei die erste Verlustgröße eine Absorptionslinie einer Wasserlösung mit einem Blutbestandteil bei der ersten Frequenz und wobei die zweite Verlustgröße eine Absorptionslinie der Wasserlösung bei der zweiten Frequenz ist.

4.  Erfassungsvorrichtung nach Anspruch 1 oder 2, wobei die erste Verlustgröße und die zweite Verlustgröße einen frequenzabhängigen Verlauf der Absorption einer Wasserlösung mit einem Blutbestandteil definieren, und wobei die erste Referenzgröße und die zweite Referenzgröße einen frequenzabhängigen Verlauf der Absorption der Wasserlösung bei einer Referenzkonzentration des Blutbestandteils definieren.

5.  Erfassungsvorrichtung nach einem der vorstehenden Ansprüche, wobei die erste Verlustgröße ein Absorptionsminimum oder ein Absorptionsmaximum in einem die erste Frequenz umfassenden ersten Frequenzbereich ist, und wobei die zweite Verlustgröße ein Absorptionsminimum oder ein Absorptionsmaximum in einem die zweite Frequenz umfassenden zweiten Frequenzbereich ist.

6. Erfassungsvorrichtung nach einem der vorstehenden Ansprüche, wobei der Blutbildparameter eine Konzentration eines Blutbestandteils, insbesondere Zuckers wie Glucose, oder Laktats oder Milchsäure oder Sauerstoffs in Blut ist.

7. Erfassungsvorrichtung nach einem der vorstehenden Ansprüche, wobei die erste Frequenz und die zweite Frequenz jeweils in einem Frequenzbereich zwischen 1 GHz und 15 GHz liegen.

8. Erfassungsvorrichtung nach einem der vorstehenden Ansprüche, wobei der Verlustdetektor (107) ausgebildet ist, die erste Verlustgröße und die zweite Verlustgröße durch eine Zweitormessung zu bestimmen.

9. Erfassungsvorrichtung nach einem der vorstehenden Ansprüche, wobei der Verlustdetektor (107) einen Netzwerkanalysator oder einen Leistungsdetektor umfasst.

10. Erfassungsvorrichtung nach einem der vorstehenden Ansprüche, wobei der Verlustdetektor (107) ausgebildet ist, zur Bestimmung der ersten Verlustgröße und der zweiten Verlustgröße jeweils einen Vorwärtstransmissionsfaktor $S_{21}$ oder einen Eingangsreflexionsfaktor $S_{11}$ zu bestimmen.

11. Erfassungsvorrichtung nach einem der vorstehenden Ansprüche, wobei der Verlustdetektor (107) ausgebildet ist, die erste Verlustgröße und die zweiten Verlustgröße jeweils auf der Basis der folgenden Formel zu bestimmen:

$$P_{Verlust} = 1 - \left| S_{11} \right|^2 - \left| S_{21} \right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

12. Erfassungsvorrichtung nach einem der vorstehenden Ansprüche, wobei der Sender (101) zum Einkoppeln des ersten Sendesignals oder des zweiten Sendesignals zumindest eine Sendeantenne, insbesondere eine Dipolantenne oder eine Rahmenantenne, umfasst, und wobei der Empfänger (105) zum Empfangen des ersten Empfangssignals und des zweiten Empfangssignals zumindest eine Empfangsantenne, insbesondere eine Dipolantenne oder eine Rahmenantenne oder eine Patch-Antenne, umfasst.

13. Erfassungsvorrichtung nach einem der vorstehenden Ansprüche, wobei der Sender (101) ausgebildet ist, das erste Sendesignal oder das zweite Sendesignal als eine Mode oder einen Wellentyp, insbesondere als eine transversal-elektrische (TE) Welle oder als eine transversal-magnetische (TM) Welle oder eine transversalelektromagnetische (TEM) Welle in das Blutgefäß einzukoppeln, insbesondere longitudinal oder transversal bezüglich eines Verlaufes des Blutgefäßes oder einer Blutflussrichtung, einzukoppeln.

14. Erfassungsvorrichtung nach einem der vorstehenden Ansprüche, wobei der Sender (101) ausgebildet ist, das erste Sendesignal und das zweite Sendesignal sukzessive, insbesondere mittels eines Sendesignalgenerators (1101) oder durchstimmbaren Oszillators (1101), oder gleichzeitig, insbesondere mittels eines das erste Sendesignal und das zweite Sendesignal umfassenden Breitbandsignals, in das Blutgefäß einzukoppeln.

15. Verfahren zum Erfassen eines Blutbildparameters von Blut in einem Blutgefäß, mit:

Einkoppeln (1501) eines ersten Sendesignals mit einer ersten Frequenz in das Blutgefäß;
Einkoppeln (1503) eines zweiten Sendesignals mit einer zweiten Frequenz in das Blutgefäß;
Empfangen (1505) eines ersten Empfangssignals bei der ersten Frequenz;
Empfangen (1507) eines zweiten Empfangssignals bei der zweiten Frequenz;
Ermitteln (1509) einer ersten Verlustgröße auf der Basis des ersten Sendesignals und des ersten Empfangssignals bei der ersten Frequenz;
Ermitteln (1511) einer zweiten Verlustgröße auf der Basis des zweiten Sendesignals und des zweiten Empfangssignals bei der zweiten Frequenz;
Bestimmen (1513) einer ersten Frequenzverschiebung der ersten Verlustgröße relativ zu einer ersten Referenzverlustgröße;
Bestimmen (1515) einer zweiten Frequenzverschiebung der zweiten Verlustgröße relativ zu einer zweiten Referenzverlustgröße; und
Bestimmen (1517) des Blutbildparameters auf der Basis der ersten Frequenzverschiebung und der zweiten Frequenzverschiebung.

**Claims**

1. Detection device for detecting a blood picture parameter of blood in a blood vessel, comprising:

a transmitter (101), which is configured to couple a first transmission signal with a first frequency and a second transmission signal with a second frequency into the blood vessel;
a receiver (105), which is configured to receive a first reception signal at the first frequency and a second reception signal at the second frequency; and

a loss detector (107), which is configured to:

determine a first loss variable on the basis of the first transmission signal and the first reception signal at the first frequency; and determine a second loss variable on the basis of the second transmission signal and the second reception signal at the second frequency; and also

a processor (109), which is **characterized in that** it is configured to:

determine a first frequency shift of the first loss variable relative to a first reference loss variable;

to determine a second frequency shift of the second loss variable relative to a second reference loss variable; and

to determine the blood picture parameter on the basis of the first frequency shift and the second frequency shift.

2. Detection device according to Claim 1, which furthermore comprises a storage medium (111) for providing the first reference loss variable and the second reference loss variable.

3. Detection device according to Claim 1, wherein the first loss variable is an absorption line of a water solution with a blood constituent at the first frequency and wherein the second loss variable is an absorption line of the water solution at the second frequency.

4. Detection device according to Claim 1 or 2, wherein the first loss variable and the second loss variable define a frequency-dependent profile of the absorption of a water solution with a blood constituent and wherein the first reference variable and the second reference variable define a frequency-dependent profile of the absorption of the water solution at a reference concentration of the blood constituent.

5. Detection device according to one of the preceding claims, wherein the first loss variable is an absorption minimum or an absorption maximum in a first frequency range comprising the first frequency and wherein the second loss variable is an absorption minimum or an absorption maximum in a second frequency range comprising the second frequency.

6. Detection device according to one of the preceding claims, wherein the blood picture parameter is a concentration of a blood constituent, in particular sugar such as glucose, or of lactate or lactic acid or of oxygen in the blood.

7. Detection device according to one of the preceding claims, wherein the first frequency and the second frequency respectively lie in a frequency range between 1 GHz and 15 GHz.

8. Detection device according to one of the preceding claims, wherein the loss detector (107) is configured to determine the first loss variable and the second loss variable by means of a two-port measurement.

9. Detection device according to one of the preceding claims, wherein the loss detector (107) comprises a network analyzer or a power detector.

10. Detection device according to one of the preceding claims, wherein the loss detector (107) is configured to determine in each case a forward transmission factor $S_{21}$ and an input reflection factor $S_{11}$ in order to determine the first loss variable and the second loss variable.

11. Detection device according to one of the preceding claims, wherein the loss detector (107) is configured to determine in each case the first loss variable and the second loss variable on the basis of the following formula:

$$P_{loss} = 1 - |S_{11}|^2 - |S_{21}|^2,$$

where $P_{loss}$ denotes the respective loss variable, and where $S_{11}$ denotes the input reflection factor and $S_{21}$ denotes the forward transmission factor.

12. Detection device according to one of the preceding claims, wherein the transmitter (101) for coupling-in the first transmission signal or the second transmission signal comprises at least one transmission antenna, in particular a dipole antenna or a frame antenna, and wherein the receiver (105) for receiving the first reception signal and the second reception signal comprises at least one reception antenna, in particular a dipole antenna or a frame antenna or a patch antenna.

13. Detection device according to one of the preceding claims, wherein the transmitter (101) is configured to couple the first transmission signal or the second transmission signal into the blood vessel as a mode or a wave type, in particular as a transverse electric (TE) wave or as a transverse magnetic (TM) wave or a transverse electromagnetic (TEM) wave, in particular longitudinally or transversely with respect to a direction of the blood vessel or to a blood flow direction.

14. Detection device according to one of the preceding claims, wherein the transmitter (101) is configured to couple the first transmission signal and the second transmission signal into the blood vessel successively, in particular by means of a transmission signal generator (1101) or tunable oscillator (1101), or simultaneously, in particular by means of a broadband signal comprising the first transmission signal and the second transmission signal.

15. Method for detecting a blood picture parameter of blood in a blood vessel, comprising the following steps:

coupling (1501) a first transmission signal with a first frequency into the blood vessel;
coupling (1503) a second transmission signal with a second frequency into the blood vessel;
receiving (1505) a first reception signal at the first frequency;
receiving (1507) a second reception signal at the second frequency;
establishing (1509) a first loss variable on the basis of the first transmission signal and the first reception signal at the first frequency;
establishing (1511) a second loss variable on the basis of the second transmission signal and the second reception signal at the second frequency;
determining (1513) a first frequency shift of the first loss variable relative to a first reference loss variable;
determining (1515) a second frequency shift of the second loss variable relative to a second reference loss variable; and
determining (1517) the blood picture parameter on the basis of the first frequency shift and of the second frequency shift.

**Revendications**

1. Dispositif d'acquisition pour acquérir un paramètre de formule sanguine du sang dans un vaisseau sanguin, comprenant :

un émetteur (101) qui est configuré pour injecter dans le vaisseau sanguin un premier signal émis ayant une première fréquence et un deuxième signal émis ayant une deuxième fréquence ;
un récepteur (105) qui est configuré pour recevoir un premier signal reçu à la première fréquence et un deuxième signal reçu à la deuxième fréquence ; et
un détecteur de pertes (107) qui est configuré pour :

déterminer une première grandeur de perte en se basant sur le premier signal émis et le premier signal reçu à la première fréquence ; et
déterminer une deuxième grandeur de perte en se basant sur le deuxième signal émis et le deuxième signal reçu à la deuxième fréquence ; ainsi

qu'un processeur (109) qui est **caractérisé en ce qu'**il est configuré pour :

déterminer un premier décalage en fréquence de la première grandeur de perte par rapport à une première grandeur de perte de référence ;
déterminer un deuxième décalage en fréquence de la deuxième grandeur de perte par rapport à une deuxième grandeur de perte de référence ; et
déterminer le paramètre de formule sanguine en se basant sur le premier décalage en fréquence et le deuxième décalage en fréquence.

2. Dispositif d'acquisition selon la revendication 1, lequel comprend en outre une mémoire (111) pour mettre à disposition la première grandeur de perte de référence et la deuxième grandeur de perte de référence.

3. Dispositif d'acquisition selon la revendication 1, avec lequel la première grandeur de perte est une ligne d'absorption d'une solution aqueuse avec une composante du sang à la première fréquence et avec lequel la deuxième grandeur de perte est une ligne d'absorption de la solution aqueuse à la deuxième fréquence.

4. Dispositif d'acquisition selon la revendication 1 ou 2, avec lequel la première grandeur de perte et la deuxième grandeur de perte définissent une courbe de l'absorption en fonction de la fréquence d'une solution aqueuse avec une composante du sang, et avec lequel la première grandeur de référence et la deuxième grandeur de référence définissent une courbe de l'absorption en fonction de la fréquence de la solution aqueuse à une concentration de référence de la composante du sang.

5. Dispositif d'acquisition selon l'une des revendications précédentes, avec lequel la première grandeur de perte est une absorption minimale ou une absorption maximale dans une première plage de fréquences incluant la première fréquence, et avec lequel la deuxième grandeur de perte est une absorption minimale ou une absorption maximale dans une deuxième plage de fréquences incluant la deuxième fréquence.

**6.** Dispositif d'acquisition selon l'une des revendications précédentes, avec lequel le paramètre de formule sanguine est une concentration d'une composante du sang, notamment le sucre tel que le glucose, ou le lactate ou l'acide lactique ou l'oxygène dans le sang.

**7.** Dispositif d'acquisition selon l'une des revendications précédentes, avec lequel la première fréquence et la deuxième fréquence se trouvent respectivement dans une plage de fréquences entre 1 GHz et 15 GHz.

**8.** Dispositif d'acquisition selon l'une des revendications précédentes, avec lequel le détecteur de pertes (107) est configuré pour déterminer la première grandeur de perte et la deuxième grandeur de perte par le biais d'une mesure à deux portes.

**9.** Dispositif d'acquisition selon l'une des revendications précédentes, avec lequel le détecteur de pertes (107) comprend un analyseur de réseau ou un détecteur de puissance.

**10.** Dispositif d'acquisition selon l'une des revendications précédentes, avec lequel le détecteur de pertes (107) est configuré, en vue de la détermination de la première grandeur de perte et de la deuxième grandeur de perte, pour déterminer à chaque fois un facteur de transmission dans le sens direct $S_{21}$ ou un facteur de réflexion d'entrée $S_{11}$.

**11.** Dispositif d'acquisition selon l'une des revendications précédentes, avec lequel le détecteur de pertes (107) est configuré pour déterminer la première grandeur de perte et la deuxième grandeur de perte à chaque fois en se basant sur la formule suivante :

$$P_{Perte} = 1 - |S_{11}|^2 - |S_{21}|^2,$$

$P_{Perte}$ désignant la grandeur de perte correspondante, $S_{11}$ le facteur de réflexion d'entrée et $S_{21}$ le facteur de transmission dans le sens direct.

**12.** Dispositif d'acquisition selon l'une des revendications précédentes, avec lequel l'émetteur (101), pour l'injection du premier signal émis ou du deuxième signal émis, comprend au moins une antenne d'émission, notamment une antenne dipôle ou une antenne cadre, et avec lequel le récepteur (105), pour la réception du premier signal reçu et du deuxième signal reçu, comprend au moins une antenne de réception, notamment une antenne dipôle ou une antenne cadre ou une antenne composite.

**13.** Dispositif d'acquisition selon l'une des revendications précédentes, avec lequel l'émetteur (101) est configuré pour injecter dans le vaisseau sanguin le premier signal émis ou le deuxième signal émis sous la forme d'un mode ou d'une type d'onde, notamment sous la forme d'une onde électrique transversale (TE) ou sous la forme d'une onde magnétique transversale (TM) ou d'une onde électromagnétique transversale (TEM), notamment pour l'injecter longitudinalement ou transversalement par rapport à un tracé du vaisseau sanguin ou d'un sens de circulation du sang.

**14.** Dispositif d'acquisition selon l'une des revendications précédentes, avec lequel l'émetteur (101) est configuré pour injecter successivement dans le vaisseau sanguin le premier signal émis et le deuxième signal émis, notamment au moyen d'un générateur de signal émis (1101) ou d'un oscillateur accordable (1101), ou simultanément, notamment au moyen d'un signal à large bande qui englobe le premier signal émis et le deuxième signal émis.

**15.** Procédé d'acquisition d'un paramètre de formule sanguine du sang dans un vaisseau sanguin, comprenant :

injection (1501) dans le vaisseau sanguin d'un premier signal émis ayant une première fréquence ;
injection (1503) dans le vaisseau sanguin d'un deuxième signal émis ayant une deuxième fréquence ;
réception (1505) d'un premier signal reçu à la première fréquence ;
réception (1507) d'un deuxième signal reçu à la deuxième fréquence ;
détection (1509) d'une première grandeur de perte en se basant sur le premier signal émis et le premier signal reçu à la première fréquence ;
détection (1511) d'une deuxième grandeur de perte en se basant sur le deuxième signal émis et le deuxième signal reçu à la deuxième fréquence ;
détermination (1513) d'un premier décalage en fréquence de la première grandeur de perte par rapport à une première grandeur de perte de référence ;
détermination (1515) d'un deuxième décalage en fréquence de la deuxième grandeur de perte par rapport à une deuxième grandeur de perte de référence ; et
détermination (1517) du paramètre de formule sanguine en se basant sur le premier décalage en fréquence et le deuxième décalage en fréquence.

103

Sender

Empfänger

101

105

Verlustdetektor

107

Speicher

Prozessor

109

111

100

Fig. 1

Fig. 2A     Fig. 2B     Fig. 2C

Fig. 3A

Fig. 3B

Fig. 3C

Fig. 3D

407

408

401

403

405

Fig. 4A

409    413

411

415

Fig. 4B

Fig. 5A

Fig. 5B

603

601

605

Fig. 6A

609

607

605

605

H

611

603

603

Fig. 6B

Fig. 7

Fig. 8

Fig. 9

Fig. 10

Fig. 11

Fig. 12

Fig. 13

| Durchm. El | A | B | C | D | E | F | G |
|------------|-----|-----|-----|-----|-----|------|-----|
| 6 mm | <1 | +2 | -1 | -9 | -17 | +11 | <1 |
| 3,4 mm | <1 | +4 | -1 | -13 | – | – | -10 |

Fig. 14

1501

| Einkoppeln |

1505

| Empfangen |

1509

| Ermitteln einer Verlustgröße |

1513

| Bestimmen einer Frequenzverschiebung |

1503

| Einkoppeln |

1507

| Empfangen |

1511

| Ermitteln einer Verlustgröße |

1515

| Bestimmen einer Frequenzverschiebung |

| Bestimmen des Blutbildparameters |

1517

Fig. 15

Fig. 16

Fig. 17

Fig. 18

Fig. 19

Fig. 20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20060025664 A1
- WO 2010131029 A1

**In der Beschreibung aufgeführte Nicht-Patentliteratur**

- **VON ANDREAS CADUFF et al.** Non-invasive glucose monitoring in patients with Type 1 diabetes: A multi-sensor system combining sensors for dielectric and optical characterization of skin. *Biosensors and Bioelectronics,* 2009, vol. 24, 2778-2784
- **VON BUFORD RANDAL JEAN et al.** A microwave frequency sensor for non-invasive blood-glucose measurement. *SAS 2008 - IEEE Sensors Applications Symposium,* 12. Februar 2008
- **M. MCCLUNG.** Calibration methodology for a microwave non-invasive glucose sensor. *Master Thesis,* Mai 2008
- **NETTER, F. N.** Atlas der Anatomie. Thieme Verlag, 2006